# EUROPEAN PATENT SPECIFICATION

(11) **EP 3 851 433 B1**
(45) Date of publication and mention of the grant of the patent: **17.12.2025**
(21) Application number: 19859050.7
(22) Date of filing: 11.09.2019
(51) Int. Cl.: C07C 51/305, C07C 61/135, C07C 213/02, C07C 213/10, C07C 219/24, C07C 231/08, C07C 231/12, C07C 233/23, C07C 233/52, C07C 269/04, C07C 269/06, C07C 271/24

(54) **PREPARATION PROCESS FOR AMANTADINE NITRATE DERIVATIVE**
VERFAHREN ZUR HERSTELLUNG VON AMANTADIN-NITRAT-DERIVAT
PROCÉDÉ DE PRÉPARATION D'UN DÉRIVÉ DE NITRATE D'AMANTADINE

(30) Priority: 12.09.2018 CN 201811060724
(43) Date of publication of application: 21.07.2021
(73) Proprietor: Qingdao Hailan Pharmaceuticals Co., Ltd., Qingdao, Shandong 266111 (CN)
(72) Inventor: WANG, Yuqiang, Qingdao, Shandong 266111 (CN); LIU, Zheng, Qingdao, Shandong 266111 (CN); SUN, Yewei, Qingdao, Shandong 266111 (CN); ZHANG, Zaijun, Qingdao, Shandong 266111 (CN); ZHANG, Gaoxiao, Qingdao, Shandong 266111 (CN)
(74) Representative: Uexküll & Stolberg
(86) International application number: PCT/CN2019/000178
(87) International publication number: WO 2020/052179

(56) References cited:
- WO-A2-2009/057140
- CN-A- 1 486 180
- CN-A- 105 294 450
- CN-A- 107 365 255
- CN-A- 109 206 317
- LIU, ZHENG ET AL.: "Synthesis and Biological Evaluation of Memantine Nitrates as a Potential Treatment for Neurodegenerative Diseases", MEDICAL CHEMESTRY COMMUNICATION, vol. 8, no. 1, 20 October 2016 (2016-10-20), pages 135 - 147, XP055692256, ISSN: 2040-2503, DOI: 10.1039/C6MD00509H

## Description

### FIELD OF THE INVENTION

The present invention relates the medical field, and relates to a process for the manufacture of medicaments, and more particularly to a process for the manufacture of amantadine nitrate derivatives.

### BACKGROUND OF THE INVENTION

Amantadine and its derivatives have varieties of biological activities and are widely used in the field of medicine. Memantine (1,3-Dimethylamantadine, Memantine) is a non-competitive antagonist of NMDA receptors, and is mainly used for the treatment of moderate to severe Alzheimer's Disease (AD). By binding to the NMDA receptor in ion channel, memantine can block influx of K⁺ and Ca²⁺ plasmas with neuroprotective effects. The binding of memantine to the NMDA receptor is reversible with a moderate dissociation rate, which can ensure the pharmacological effects and also prevent from being accumulated in the channel to affect the normal physiological functions of the receptor (Lipton et al., Journal of neurochemistry. 2006, 97: 1611-1626). Also, memantine, which binding to the NMDA receptor is voltage-dependent, can only bind to the receptor when the neuron is depolarized, and thus can block the activation of the NMDA receptor when the neuron continues to be depolarized under pathological conditions, but would not block the activation of NMDA receptor under normal physiological conditions (Wenk et al., CNS drug reviews. 2003, 9(3): 275-308; McKeage., Drugs & Aging. 2010, 27(2): 177-179).

Nitric oxide (NO) is indicated to have varieties of in vivo biological activities. As a free radical gas, NO has an unpaired electron with extremely unstable chemical properties and is very easy to combine with free radicals, and thereby reduces the number of free radicals and reduces the harm caused by oxidative damage to body tissues. In addition, as a signaling molecule, NO also plays multiple roles in the cardiovascular system. Endogenous NO is a vasodilator, which acts on guanylate cyclase in vascular smooth muscle cells to promote vasodilation and lower blood pressure. Also, it can enter platelet cells, reduce their activity, thereby inhibit their aggregation and adhesion to the vascular endothelium, prevent thrombosis, and prevent atherosclerosis. Small molecule drugs that can release NO, such as nitroglycerin, sodium nitroprusside, isosorbide mononitrate, are widely used for the treatment of many clinical diseases.

Amantadine nitrate compound (II) is a NO donating small molecule compound (CN105294450) independently designed and developed by the inventors of this patent application: 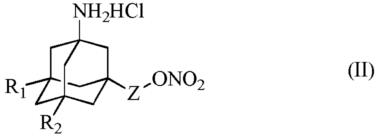 wherein R₁ and R₂ are each independently hydrogen, straight-chain or branched-chain alkyl, substituted or unsubstituted aryl or heteroaryl, substituted or unsubstituted esters, substituted amines; and Z straight- or branched-carbon chain with 0 to 6 carbon atoms connecting to the nitrate ester group and can be substituted with a heteroatom, alkyl group, aryl group, and aryl hetero group. Preferably, the alkyl group is a C1-C10 alkyl group.

Preliminary pharmacological studies have found that these compounds can play a similar role to memantine in effectively antagonizing the excessive activation of NMDA receptors caused by glutamate, inhibiting the influx of calcium ions, and scavenging free radicals in the cerebral cortex, and thereby effectively protect cerebral cortex neurons, and, on the other hand, it can release NO efficiently and relax arteries (Liu et al., Med. Chem. Comm., 2017, 8, 135-147).

Such compounds have shown good therapeutic effects in rat models for various diseases of nervous system or neurovascular system. Among them, the compound MN-08 (III) can significantly reduce the cerebral infarction area and cerebral edema in rats caused by ischemic stroke; significantly improve the memory impairment and behavioral impairment of vascular dementia rats; and also reduce monocrotaline-induced pulmonary artery pressure in rats with pulmonary hypertension, and inhibit pulmonary artery remodeling and right ventricular hypertrophy. In addition, MN-08 is indicated to have a good effect on rats with subarachnoid hemorrhage, glaucoma and other disease models.

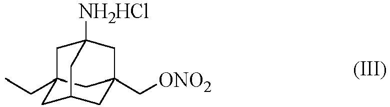

Therefore, as a new candidate class one drug molecule with completely independent intellectual property rights, the MN-08 and amantadine nitrate derivatives have broad development prospects and great practical and economic significance.

In the early stage, the inventors of the subject application explored a laboratory small-scale synthetic method of these compounds (CN105294450). In the method, substituted or unsubstituted adamantane was used as the raw material, which was brominated and hydrolyzed to obtain an adamantane alcohol. The adamantane alcohol undergoes Ritter reaction to give adamantanoic acid, and further undergoes Koch-Haaf reaction to give acetamidoadamantic acid. In the compound the carboxyl group is reduced to obtain an acetamidoadamantane methanol. To the acetamidoadamantane methanol with diethylene glycol (15-20 Vol) as the solvent was added a high dose of sodium hydroxide for hydrolysis at high temperature. The hydrolyzed product with tetrahydrofuran as solvent was condensed with Boc anhydride under conditions of triethylamine and DMAP. The condensation product was nitrated with a nitrating reagent mixed with fuming nitric acid and acetic anhydride, and finally the Boc group was removed with HCl to obtain amantadine nitrate compound as shown in the following scheme. 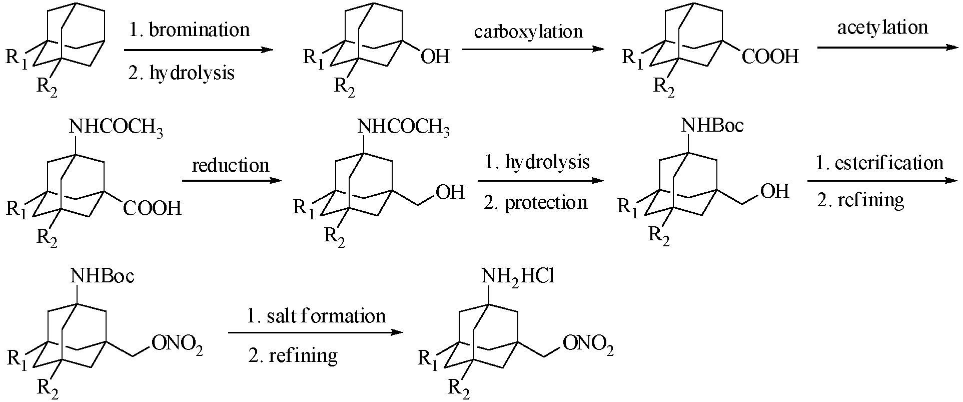 wherein R₁ and R₂ are each independently hydrogen, straight-chain or branched-chain alkyl, substituted or unsubstituted aryl or heteroaryl.

In the above synthetic method, the dosage ratio of the reagents and solvents is not optimized. The usage amount of the reaction reagents such as bromine, sodium hydroxide, and strong acid and strong base, such as sulfuric acid and nitric acid, is too high, which caused waste in reagents, increased costs, and added burden on environmental protection; moreover, the purification of the compounds after each step of the reaction was obtained through separation and purification with silica gel column, which is not suitable for industrial batch production.

More importantly, during the research process of the present invention, the inventors conducted research and development of a scale-up pilot process described herein below, and discovered the above-mentioned limitations of the previous laboratory small-scale synthesis method. The inventors discovered through scale-up pilot tests of ethyl-adamantane nitrate that, in the nitration reaction, when the reaction was run by using the relative amount of the materials in the laboratory small scale process, with the nitrifying agent fuming nitric acid and acetic anhydride being in the ratio of Starting Materials : Ac₂O : HNO₃ = 1 : 1.8 : 2.8, the reaction speed was found very fast, and the reactants were easily reacted into completion and quickly turned into side products (III), mostly being transformed into side products (1.5 h) within the reaction time, and subsequently the yield of nitrate esters was reduced from 60-70% to about 18%, which ultimately resulted in failure of the production process with impossible separation and purification of nitrate ester intermediates. Therefore, in the previous process, the usage amount of the nitrating reagent is not suitable for industrial production.

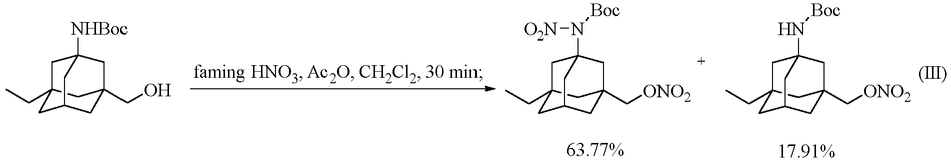

### SUMMARY OF THE INVENTION

The present invention is directed to solve the technical problems in a previous process and achieve optimization of the process conditions with significant reduction in the usage of various reaction reagents such as strong acids and strong bases, energy consumption, and costs of production; and also to provide an optimized post-treatment process suitable for industrial production; and further, more importantly, to adjust the amount of fuming nitric acid and acetic anhydride in the nitrating reagent, to find the appropriate process dosage and reaction rate to meet the requirements of production process, and to reduce the generation of by-products with simplified post-treatment process.

The purpose of the present invention is to provide a cost effective, green, safe and reliable industrial process for manufacture of amantadine nitrate derivatives.

The process for manufacture of amantadine nitrate derivatives of the present invention is as defined in claim 1 and includes the following steps: (1) synthesis of adamantanol from adamantane; (2) carboxylation of adamantanol; (3) acetylation of adamantanoic acid; (4) reduction; (5) hydrolysis of amido adamantanol and Boc protection of amino group; (6) crystallization of Boc protected amantadinol; (7) nitrate esterification of Boc protected amantadinol; (8) refining of product of nitrate esterification; (9) Boc deprotection and salt formation; and (10) refining of amantadine nitrate hydrochloride: 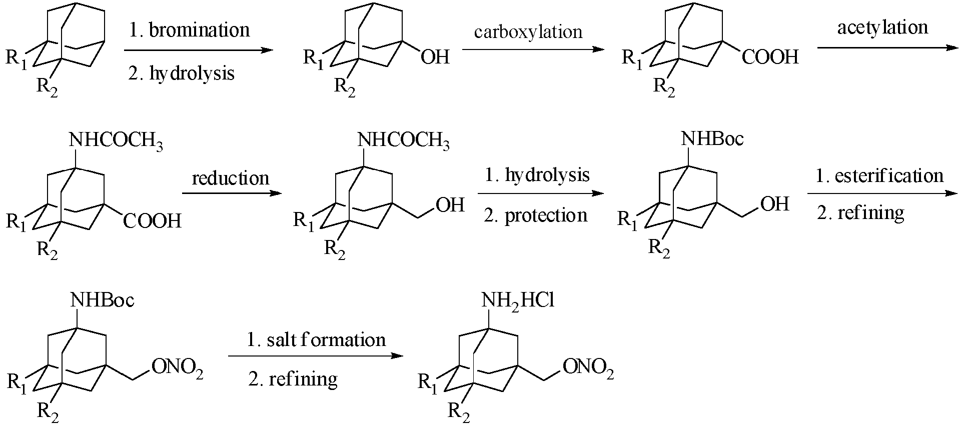 wherein, R₁ and R₂ are each independently hydrogen, straight-chain or branched-chain alkyl, substituted or unsubstituted aryl or heteroaryl.

In the above process of manufacture, the raw material adamantane has the structure (A): 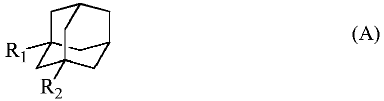 wherein R₁ and R₂ are each independently hydrogen, straight-chain or branched-chain alkyl, substituted or unsubstituted aryl or heteroaryl.

In various embodiments of the invention, the process of manufacture may include the following technical features.

In the process of manufacture, step (1) further includes following reactions:
(1a) Bromination of adamantane: reacting a substituted or unsubstituted adamantane with liquid bromine in reflux for 4-6 h to obtain bromoadamantane; and
(1b) Hydrolysis of bromoadamantane: adding sodium oxalate and water, the reaction system being refluxed at 75 °C via hydrolysis to obtain a substituted or unsubstituted adamantanol.

In the process of manufacture, step (2) further comprises: the product from step (1) being dissolved in formic acid, and slowly added dropwise to concentrated sulfuric acid with cooling to 0-10 °C, and then the reaction being maintained at 0-10 °C for 3-6 h; the reaction solution being poured into ice water and stirred for 16-18 h; after suction filtration, the solid filter cake being washed with water, dissolved with 0.27X sodium hydroxide solution, and filtered with suction; the filtrate being acidified with 5-10% hydrochloric acid to pH=1-2 and filtered, the filter cake being washed with water until the filtrate being neutral, and dried at 40-50 °C to obtain adamantanoic acid.

In the process of manufacture, step (3) further comprises: cooling concentrated sulfuric acid to 0-10 °C, and adding adamantanoic acid to be dissolved with stirring, adding slowly dropwise nitric acid, and then adding dropwise acetonitrile, reaction being maintained at 0-10 °C for 2-3 h; reaction solution being poured into ice water with stirring for 16-18 h, and filtered with suction, and filter cake being washed with water and dried at 40-50 °C to obtain amido adamantanic acid.

In the process of manufacture, step (4) further comprises: adding the product of step (3) to tetrahydrofuran, cooled to 0-10 °C, and adding triethylamine in batches, then adding dropwise ethyl chloroformate, and reaction being run at room temperature for 4-6 h; filtering resulting reaction mixture, and washing filter cake with tetrahydrofuran, combing tetrahydrofuran phases and cooling to 0-10 °C, adding sodium borohydride in batches, then adding 0.8-1X water dropwise, and reaction being run for 2-3 h; adding 5X water, filtering reaction mixture, filtrate being spin-dried, then extracted with ethyl acetate, and combined ethyl acetate phases being dried over sodium sulfate, filtered, and spin dried, and residue being added with 1.5-2.5X ethyl acetate, stirred well, the same amount of petroleum ether being added, and stirred for 12-16 h, filtered and filter cake dried at 40-50 °C to obtain an amido adamantanol.

In the process of manufacture, step (5) further comprises following reactions:
(5a) Hydrolysis: adding amido adamantanol and a strong base sequentially to polyethylene glycol with a 5-10 times volume, reacted via high temperature hydrolysis at 100-180 °C for 10-18 h, and reaction system being cooled, diluted with purified water and stirred evenly, and water layer being extracted with toluene, dried over sodium sulfate and filtered, and the filtrate being concentrated;
(5b) Condensation: dissolving the product obtained in reaction (5a) in dichloromethane, adding Boc anhydride, then running reaction via condensation at 20-30 °C for 1-5 h, and resulting mixture being concentrated to remove dichloromethane.

In the process of manufacture, step (6) further comprises: adding n-Hexane to resulting residual oily liquid of step (5), reacted via crystallization for 2-3 h, and the resulting mixture being centrifuged, washed with n-hexane, and then dried at 40-50 °C for 16-18 h to obtain Boc-protected amantadinol.

In the process of manufacture, step (7) further comprises: mixing fuming nitric acid with acetic anhydride at a temperature of -10 °C to 10 °C to give a nitrating reagent; dissolving resulting solid product from step (6) in dichloromethane with temperature controlled at -10°C to 10°C, adding dropwise the nitrating reagent to be reacted for 15 min to 6 h, then quenching reaction upon its completion with ice water and water layer being separated, and organic layer being washed respectively with 10 times volume (10 Vol) of a solution of saturated sodium bicarbonate and a solution of saturated sodium chloride, dried over sodium sulfate, filtered, and concentrated to remove dichloromethane to obtain a crude product of Boc-protected amantadine nitrate

In the process of manufacture, step (8) further comprises: dissolving the crude product of step (7) in alcohol, adding water for crystallization with mashing for 2-3 h, then the resulting mixture being filtered, and resulting solid being dried at 40-50 °C to obtain a refined product.

In the process of manufacture, step (9) further comprises: dissolving the product of step (8) in ethyl acetate with temperature controlled at 10-30 °C, adding HCl/ethyl acetate, running reaction for 16-18 h, and then resulting mixture being filtered with suction, and dried to obtain an amantadine nitrate hydrochloride.

In the process of manufacture, step (10) further comprises: dissolving product of step (9) in ethanol, treating via filtration with filtrate being concentrated, and adding ethyl acetate for crystallization with beating, resulting mixture being dried to constant weight to obtain a refined amantadine nitrate hydrochloride.

In the process of manufacture, in reaction (1a), the molar ratio of reaction materials is raw material : bromine = 1 : 2-6, reaction temperature is 60-90 °C, and reaction time is 4-6 h;
In the process of manufacture, in reaction (1b), the molar ratio of reaction materials is raw material : sodium oxalate = 1 : 2.5-6.0, the amount of water used is 23-35X, and reaction time is 2-3 h.

In the process of manufacture, step (1), the post-treatment of step (1) comprises: adding 3-5X sodium sulfite solution to reaction solution, stirring at room temperature for 16-18 h and then filtered, and filter cake being washed with water and then dissolved with 3-8X organic solvent, dried over anhydrous sodium sulfate, and filtered, and filter cake being washed with 2-5X organic solvent, and spin-dried under reduced pressure to obtain adamantanol as a solid; wherein, the organic solvents are preferably methyl tert-butyl ether, ethyl acetate, dichloromethane and the like.

In the process of manufacture, step (2), the molar ratio of reaction feed is raw material : formic acid : sulfuric acid = 1 : 6-10 : 11-16, wherein the molar ratio of reaction feed is preferably raw material : formic acid : sulfuric acid = 1 : 6-8 : 11.5-13, and the reaction time is preferably 4-5 h.

In the process of manufacture, step (3), the molar ratio of reaction feed is raw material : nitric acid : sulfuric acid : acetonitrile = 1 : 1.6-2.5 : 9-15 : 1.1-1.5.

In the process of manufacture, step (4), the molar ratio of reaction feed is raw material : triethylamine : ethyl chloroformate : sodium borohydride = 1 : 1.2-1.5 : 1.2-1.5 : 1.5-2.5.

In the process of manufacture, step (5), the strong base is lithium hydroxide, sodium hydroxide, or potassium hydroxide, and the molar ratio of reaction feed is raw material : base = 1 : 5-10.

In the process of manufacture, step (5), the reaction temperature is 100-180 °C, and the reaction time is preferably 15-16 h.

In the process of manufacture, step (5), the amount of dichloromethane is 5-10 times the volume (5-10 Vol) of the raw material, and the molar ratio of reaction feed is raw material : Boc anhydride = 1 : 1.05-1.5, and the reaction time is 1-5 h.

In the process of manufacture, step (6), the amount of n-hexane used for crystallization of the product after condensation is 5-9 times in volume (5-9 Vol) of the raw material.

In the process of manufacture, step (7), the molar ratio of reaction fee is raw material : acetic anhydride : fuming nitric acid = 1 : 1.08-1.8 : 1.68-2.8.

In the process of manufacture, step (7), the nitrating reagent is prepared at -10-10 °C, and then stirred for 0.5-1 h.

In the process of manufacture, step (7), the reaction temperature is 0-10 °C, and the reaction time is 15 min-6 h, preferably 30 min-3 h.

In the process of manufacture, step (8), the alcohol can be methanol or ethanol, and the weight ratio of reaction feed is raw material : alcohol 1: water = 1 : 2-5 : 3-8.

In the process of manufacture, step (8), after the oil obtained from step (7) is completely dissolved in ethanol, a portion of water is added with stirring until precipitation of a white solid, stirred for 0.5-1 h, and then the remaining water is added with beating for 1-2 h.

In the process of manufacture, step (8), the crystallization temperature is controlled at 20-30 °C.

In the process of manufacture, step (9), the concentration of HCl in HCl/ethyl acetate is 2.0-4.37 M.

In the process of manufacture, step (9), the molar ratio of raw materials : HCl = 1 : 5-10, the reaction temperature is 20-30 °C, and the reaction time is 16-18 h.

In the process of manufacture, step (9), the refined compound in step (8) is first dissolved in 3-6 times the weight of ethyl acetate, and then HCl/ethyl acetate is added for the reaction.

In the process of manufacture, step (10), the crude material is dissolved in alcohol, then concentrated under reduced pressure to 0.5-1 times the remaining ethanol in the system, and ethyl acetate is added with beating for 2-3 h.

In the process of manufacture, step (10), the weight ratio of the reaction feed is raw material : ethanol : ethyl acetate = 1 : 2.5-3 : 15-25.

In the process of manufacture, step (10), the temperature of ethanol for concentration is controlled at 40-50 °C, and the drying temperature is controlled at 45-50 °C.

As compared with the prior technology, the process of the present invention has significant advantages, including:
1) Through the optimization of the process, the amount of chemical reagents such as bromine, strong acids, strong bases used in the process was reduced, and the production cost was reduced. The waste liquids generated in the post-treatment and the production processes were reduced, and thus the process becomes more environmentally friendly;
2) In the step of hydrolysis, polyethylene glycol 400 is selected as the reaction solvent, which lowered the reaction temperature, reduced energy consumption, and made the process more environmentally friendly;
3) In the nitrification reaction, by controlling the amount of fuming nitric acid, the reaction speed and reaction time were adjusted, which reduced the formation of by-products, increased the output of the main product, and made the process more suitable for industrial production; furthermore, through the refining process, the post-treatment process was simplified, and the amount of other organic reagents was reduced;
4) In the salt-forming reaction, the amount of HCl is reduced, the refining process is simplified, and the impurities after washing are reduced, and thus the process is more environmentally friendly and suitable for industrial production.

Generally, the process of the present invention reduced the amount of reaction reagents in each step and reduced production costs. Also, the processes such as crystallization and filtration were used in the post-treatment, and the column chromatography separation and purification in the laboratory process were abandoned, and the purification process in post-treatment was simplified. More importantly, the process of the present invention suspended the nitrification reaction speed through optimization in the amount of nitrating reagents and appropriate extension of reaction time, and thus the conversion of main products to by-products is significantly reduced within 30 min-6 h, which can meet the requirements of time control in industrial production, and increase yield, reduce by-products, and make the overall process more green and environmentally friendly, with great industrial and socio-economic values. Therefore, the process for manufacture of the present invention has the advantages of low production costs, improved safe and reliability, and high reaction yields, and is very suitable for industrial production.
The following examples are used to further explain the present invention.

### DETAILED DESCRIPTION OF THE EMBODIMENTS

In the following embodiments, the process for manufacture of compound MN-08 will describe in more detail as examples of the technologies of the invention. Obviously, they are shown as only a part but not all of the embodiments of the present invention.

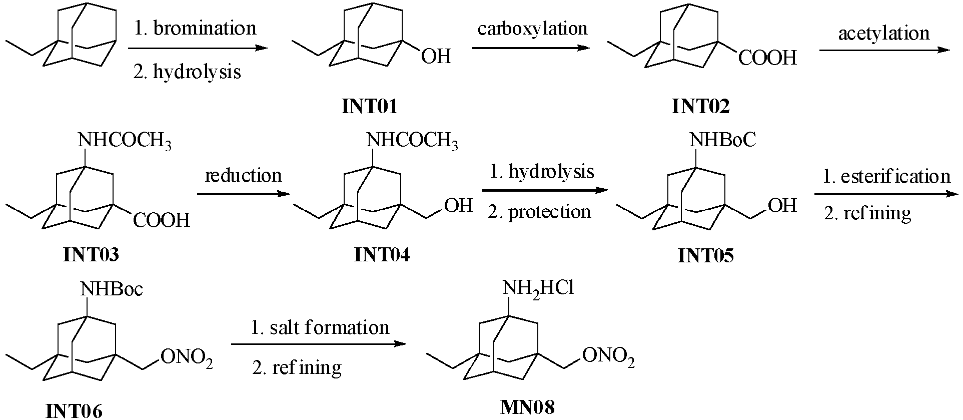

### Example 1. Preparation of INT01

In a 2 L round bottom flask was placed 100 g (0.61 mol) of ethyladamantane, 495 g (5X, 2.9 mol) of Br₂ was added, and the reaction was refluxed at 60 °C for 4 hours. The reaction solution was cooled to room temperature, and 500 g (5X, 3.6 mol) of (COONa)₂ and 800 mL (8X) of water were added, then heated to 75 °C and refluxed for 2 h. The reaction was monitored via TLC. After the reaction was completed, the reaction solution was cooled to room temperature, and then was added into a solution of saturated sodium sulfite and stirred for 16 h. The resulting mixture was filtered with suction, and the filter cake was washed with water and dissolved in 400 g (4X) of ethyl acetate, which was dried over 100 g (1X) of anhydrous sodium sulfate. After filtration, the filter residue was washed with 200 g of ethyl acetate, and the combined ethyl acetate layers were spin-dried under reduced pressure to give 102 g of INT01 as a solid with a yield of 92%.

### Example 2. Preparation of INT01

In a 2 L round bottom flask was placed 100 g (0.61 mol) of ethyladamantane, 307 g (3X, 1.8 mol) of Br₂ was added, and the reaction was refluxed at 75 °C for 4 hours. The reaction solution was cooled to room temperature, and 400 g (4X, 2.4 mol) of (COONa)₂ and 640 mL (6.4X) of water were added, then heated to 75 °C and refluxed for 2 h. The reaction was monitored via TLC. After the reaction was completed, the reaction solution was cooled to room temperature, and then was added into a solution of saturated sodium sulfite and stirred for 16 h. The resulting mixture was filtered with suction, and the filter cake was washed with water and dissolved in 400 g (4X) of ethyl acetate, which was dried over 100 g (1X) of anhydrous sodium sulfate. After filtration, the filter residue was washed with 200 g of ethyl acetate, and the combined ethyl acetate layers were spin-dried under reduced pressure to give 98 g of INT01 as a solid with a yield of 89%.

### Example 3. Preparation of INT01

In a 20 L reaction kettle was placed 1500 g (9.1 mol) of ethyladamantane, 4665 g (3.1X, 29.3 mol) of Br₂ was added, and the reaction was refluxed at 60°C for 4 hours. The reaction solution was cooled to room temperature, and 3000 g (2X, 22.3 mol) of (COONa)₂ and 3000 mL (2X) of water were added, then heated to 75 °C and refluxed for 2 h. The reaction was monitored via TLC. After the reaction was completed, the reaction solution was cooled to room temperature, and then was added into a solution of saturated sodium sulfite (4500 g, 3X) and stirred for 16 h. The resulting mixture was filtered with suction, and the filter cake was washed with water and dissolved in 6000 g (4X) of ethyl acetate, which was dried over 1000 g (0.66X) of anhydrous sodium sulfate. After filtration, the filter residue was washed with 2000 g of ethyl acetate, and the combined ethyl acetate layers were spin-dried under reduced pressure to give 1500 g of INT01 as a solid with a yield of 91%.

### Example 4. Preparation of INT01

In a 500 L reaction kettle was placed 44 kg (269 mol) of ethyladamantane, 141 kg (3.2X, 542.7 mol) of Br₂ was added, and the reaction was refluxed at 60°C for 4 hours. The reaction solution was cooled to room temperature, and 90 kg (2.04X, 3.6 mol) of (COONa)₂ and 112 L (2.55X) of water were added, then heated to 75 °C and refluxed for 2 h. The reaction was monitored via TLC. After the reaction was completed, the reaction solution was cooled to room temperature, and then was added into a solution of saturated sodium sulfite solution (135.0 kg, 3.05X) and stirred for 16 h. The resulting mixture was filtered with suction, and the filter cake was washed with water and dissolved in 343 kg (7.8X) of methyl tert-butyl ether,, which was dried over 22 kg (0.5X) of anhydrous sodium sulfate. After filtration, the filter residue was washed with 88 kg (2X) of ethyl acetate and the combined organic layers were spin-dried under reduced pressure to obtain 45 kg of INT01 as a solid with a yield of 93%.

### Example 5. Preparation of INT02

In a 1 L round bottom flask was placed 400 mL (7.4X, 13 eq) of concentrated sulfuric acid, which was cooled to 0-10 °C in an ice water bath. In 140 mL (1.7X, 6.6 eq) of formic acid was dissolved with 100 g (0.56 mol) of INT01, and the mixture was then slowly dripped into the sulfuric acid, and after the addition, the reaction was run at 0-10 °C for 4 h. The reaction was monitored via TLC. After the reaction was completed, the reaction solution was slowly added dropwise to 2 L (20X) of ice water and stirred for 16 h. The resulting mixture was filtered with suction, and the filter cake was washed ice water until pH being neutral. The filter cake was resuspended in a solution of 27 g (0.27X, 1.2 eq) of sodium hydroxide in 600 mL (6X) of water, and then filtered, and the filter residue was washed with water. The aqueous phases were combined, added with water to be diluted to 1 L, and acidified with 10% HCl to pH=1-2, and then stirred for 4-5 h. After suction filtration, the filter cake was washed with water until pH being neutral, and dried at 40-50 °C to obtain 102 g of INT02 as a white solid with a yield of 90%.

### Example 6. Preparation of INT02

In a 5 L three-necked flask was placed 4360 g (8.7X, 16 eq) of concentrated sulfuric acid, which was cooled to 0-10 °C in an ice water bath. In 1000 g (2X, 7.8 eq) formic acid was dissolved with 500 g (2.78 mol) of INT01, and the mixture was then slowly dripped into the sulfuric acid, and after the addition, the reaction was run at 0-10 °C for 6 h. The reaction was monitored via TLC. After the reaction was completed, the reaction solution was slowly added dropwise to 20 L (40X) of ice water and stirred for 16 h. The resulting mixture was filtered with suction, and the filter cake was washed ice water until pH being neutral. The filter cake was resuspended in a solution of 133 g (0.27X, 1.2 eq) of sodium hydroxide in 5000 L (10X) of water, and then filtered, and the filter residue was washed with water. The aqueous phases were combined, added with water, and acidified with 10% HCl to pH=1-2, and then stirred for 4-5 h. After suction filtration, the filter cake was washed with water until pH being neutral, and dried at 40-50 °C to obtain 485 g of INT02 as a white solid with a yield of 84%.

### Example 7. Preparation of INT02

In a 20 L reaction kettle was placed 6400 g (6.4X, 11.5 eq) of concentrated sulfuric acid, which was cooled to 0-10 °C in an ice water bath. In 1700 g (1.7X, 6.6 eq) of formic acid was dissolved with 1000 g (5.6 mol) of INT01, and the mixture was then slowly dripped into the sulfuric acid, and after the addition, the reaction was run at 0-10 °C for 4 h. The reaction was monitored via TLC. After the reaction was completed, the reaction solution was slowly added dropwise to 20 L (20X) of ice water and stirred for 16 h. The resulting mixture was filtered with suction, and the filter cake was washed ice water until pH being neutral. The filter cake was resuspended in a solution of 270 g (0.27X, 1.2 eq) of sodium hydroxide in 10 L (10X) of water, and then filtered, and the filter residue was washed with water. The aqueous phases were combined, added with water, and acidified with 10% HCl to pH=1-2, and then stirred for 4-5 h. After suction filtration, the filter cake was washed with water until pH being neutral, and dried at 40-50 °C to obtain 980 g of INT02 as a white solid with a yield of 85%.

### Example 8. Preparation of INT02

In a 500 L reaction kettle was placed 288 kg (6.4X, 11.5 eq) of concentrated sulfuric acid, which was cooled to 0-10 °C in an ice water bath. In 77 kg (1.7X, 6.6 eq) of formic acid was dissolved with 45 kg (250 mol) of INT01, and the mixture was then slowly dripped into the sulfuric acid, and after the addition, the reaction was run at 0-10 °C for 4 h. The reaction was monitored via TLC. After the reaction was completed, the reaction solution was slowly added dropwise to 900 L (20X) of ice water and stirred for 16 h. The resulting mixture was filtered with suction, and the filter cake was washed ice water until pH being neutral. The filter cake was resuspended in a solution of 12.15 kg (0.27X, 1.2 eq) of sodium hydroxide in 250 kg (5.56X) of water, , and then filtered, and the filter residue was washed with water. Concentrated hydrochloric acid was diluted from 35.1 kg to 185 kg, and was slowly added dropwise to the filtrate, and stirred for 4-5 h. After suction filtration, the filter cake was washed with water until pH being neutral, and dried at 40-50 °C to obtain 45.7 kg of INT02 as a white solid with a yield of 88%.

### Example 9. Preparation of INT03

In a 1 L three-necked flask in an ice water bath with cooling, was placed 75 mL of nitric acid (1.1X, 2.2 eq), which was then cooled to 0-10 °C in an ice water bath. To the nitric acid was added 100 g (0.48 mol) of INT02, and stirred well. To the reaction solution was slowly added dropwise 350 mL (6.4X, 13 eq) of concentrated sulfuric acid, and after the addition, the reaction was run with an ice-water bath for 1 h. Then, to the reaction solution was slowly added dropwise 31 mL (0.24X, 1.2 eq) of acetonitrile, and the reaction was continued to run with an ice water bath for 1 h. The reaction was monitored via TLC. After the reaction was complete, the reaction solution was poured into 2000 mL of ice-water mixture and stirred overnight. After suction filtration, the filter cake was washed with water until the filtrate being neutral and dried to obtain 107 g of INT03 as a white solid with a yield of 83.9%.

### Example 10 Preparation of INT03

In a 5 L three-necked flask in an ice water bath with cooling, was placed 137.5 mL of nitric acid (0.78X, 1.7 eq), which was then cooled to 0-10 °C in an ice water bath. To the nitric acid was added 250 g (1.2 mol) of INT02, and stirred well. To the reaction solution was slowly added dropwise 1700 mL (12X, 27 eq) of concentrated sulfuric acid, and after the addition, the reaction was run at 0-10 °C for 1 h. Then, to the reaction solution was slowly added dropwise 125 mL (0.4X, 2 eq) of acetonitrile, and the reaction was continued to run at 0-10 °C for 1 h. The reaction was monitored via TLC. After the reaction was complete, the reaction solution was poured into 5000 mL of ice-water mixture and stirred overnight. After suction filtration, the filter cake was washed with water until the filtrate being neutral and dried to obtain 270 g of INT03 as a white solid with a yield of 84.7%.

### Example 11. Preparation of INT03

To a 20 L reaction kettle, turned on with cooling, was placed 500 mL (0.7X, 1.6 eq) of nitric acid, which was then cooled to 0-10 °C in an ice water bath. To the nitric acid was added 1000 g (4.8 mol) of INT02, and stirred well. To the reaction solution was slowly added dropwise 3500 mL (6.4X, 13 eq) of concentrated sulfuric acid, and after the addition, the reaction was run at 0-10 °C for 1 h. Then, to the reaction solution was slowly added dropwise 310 mL (0.24X, 1.2 eq) of acetonitrile, and the reaction was continued to run at 0-10 °C for 1 h. The reaction was monitored via TLC. After the reaction was complete, the reaction solution was poured into 2000 mL of ice-water mixture and stirred overnight. After suction filtration, the filter cake was washed with water until the filtrate being neutral and dried to obtain 1050 g of INT03 as a white solid with a yield of 82.4%.

### Example 12. Preparation of INT04

To 356 g (3.56X) of tetrahydrofuran was added 100 g (0.38 mmol) of INT03 with cooling in an ice water bath, and 76 g (0.76X, 0.76 mol, 2 eq) of triethylamine was added in batches. After the solid was completely dissolved, to the solution was added dropwise 82 g of ethyl chloroformate (0.82X, 0.76 mol, 2 eq). After the addition, the ice bath was removed, and the reaction was run at room temperature for 4 h. The reaction was monitored via TLC. The reaction solution was suction filtered, and the solid filter cake was washed twice with tetrahydrofuran (1X) and then the tetrahydrofuran collections were combined. To the reaction solution was added 43 g (0.43X, 1.14 mol, 3 eq) of NaBH₄ in batches, and after the addition, 100 mL of water was slowly added and the reaction was run for 1 h. The reaction was quenched by slowly adding 300 mL of water. After the solvent was evaporated under reduced pressure, the aqueous layer was extracted with ethyl acetate (4 × 100 mL). The extract was washed twice with 100 mL of saturated NaCl solution and dried with anhydrous Na₂SO₄. The resulting material was filtered and evaporated to remove the solvent under reduced pressure to obtain a crude oil. The oil was dissolved in 200 mL of ethyl acetate, and then 200 mL of petroleum ether was added, and the mixture was stirred for 16 h. After suction filtration, the filter cake was washed with 100 mL of a mixed solution of petroleum ether : ethyl acetate = 1:1, and then dried at 40-50 °C to obtain 65 g of INT04 with a yield of 68.6%.

### Example 13, Preparation of INT04

To 890 g (3.56X) of tetrahydrofuran was added 250 g (0.94 mmol) of INT03 with cooling in an ice water bath, and 142 g (0.57X, 1.41 mol, 1.5 eq) of triethylamine was added in batches. After the solid was completely dissolved, to the solution was added dropwise 152 g (0.61X, 1.41 mol, 1.5 eq) of ethyl chloroformate. After the addition, the ice bath was removed, and the reaction was run at room temperature for 4 h. The reaction was monitored via TLC. The reaction solution was suction filtered, and the solid filter cake was washed twice with tetrahydrofuran (1X) and then the tetrahydrofuran collections were combined. To the reaction solution was added 71 g (0.28X, 1.88 mol, 2 eq) of NaBH₄ in batches, and after the addition, 200 mL of water was slowly added dropwise and the reaction was run for 1 h. The reaction was quenched by slowly adding 500 mL of water. After the solvent was evaporated under reduced pressure, the aqueous layer was extracted with ethyl acetate (4 × 250 mL). The extract was washed twice with 250 mL of saturated NaCl solution and dried with anhydrous Na₂SO₄. The resulting material was filtered and evaporated to remove the solvent under reduced pressure to obtain a crude oil. The oil was dissolved in 500 mL of ethyl acetate, and then 500 mL of petroleum ether was added, and the mixture was stirred for 16 h. After suction filtration, the filter cake was washed with 250 mL of a mixture solution of petroleum ether : ethyl acetate = 1:1, and then dried at 40-50 °C to obtain 165 g of INT04 with a yield of 70%.

### Example 14, Preparation of INT04

To 3560 g (3.56X) of tetrahydrofuran was added 1000 g (3.8 mmol) of INT03 with cooling in an ice water bath, and 460 g (0.46X, 4.65 mol, 1.2 eq) of triethylamine was added in batches.to the solution in batches. After the solid was completely dissolved, to the solution was added dropwise 502 g (0.5X, 4.65 mol, 1.2 eq) of ethyl chloroformate. After the addition, the ice bath was removed, and the reaction was run at room temperature for 4 h. The reaction was monitored via TLC. The reaction solution was suction filtered, and the solid filter cake was washed twice with tetrahydrofuran (1X) and then the tetrahydrofuran collections were combined. To the reaction solution was added 230 g (0.23X, 6.08 mol, 1.6 eq) of NaBH₄ in batches, and after the addition, 800 mL of water was slowly added dropwise and the reaction was run for 1 h. The reaction was quenched by slowly adding 3000 mL of water. After the solvent was evaporated under reduced pressure, the aqueous layer was extracted with ethyl acetate (4×1000 mL). The extract was washed twice with 1000 mL of saturated NaCl solution and dried with anhydrous Na₂SO₄. The resulting material was filtered and evaporated to remove the solvent under reduced pressure to obtain a crude oil. The oil was dissolved in 2000 mL of ethyl acetate, and then 2000 mL of petroleum ether was added, and the mixture was stirred for 16 h. After suction filtration, the filter cake was washed with 1000 mL of a mixture solution of petroleum ether : ethyl acetate = 1:1, and then dried at 40-50 °C to obtain 680 g of INT04 with a yield of 71.8%.

### Example 15. Preparation of INT05

(1) In a 5 L round bottom flask were placed 250 g (1 mol) of INT04 and 320 g (8 mol, 8 eq) of solid sodium hydroxide, and 1250 mL (5 Vol) of PEG-400 was added and refluxed at 120-130 °C for 16 h. The reaction was monitored via HPLC until INT04<0.5%, and then cooled to 70-80 °C. To the reaction was added 2.5 L of water (10 Vol), and well stirred. The resulting material was extracted once with 2.5 L (10 Vol) of toluene, and then extracted twice with toluene (1250 mL, 5 Vol). The toluene collections were combined, and washed with saturated sodium chloride solution (2.5 L, 10 Vol), and dried with anhydrous sodium sulfate. After filtration, the filter residue was washed with toluene (250 mL, 1 Vol), and the solvent was evaporated under reduced pressure to obtain 223 g of gray crude solid.
(2) In 1.3 L of dichloromethane (6 Vol) was dissolved 223 g of the crude solid, and 229 g (1.05 mol, 1.05 eq) of Boc anhydride was added, and the reaction was run at 20-30 °C with stirring for 2 h, with HPLC monitoring until INT04<0.5%. After the reaction, the dichloromethane was evaporated under reduced pressure, and to the residue was added 1.6 L of n-hexane for crystallization for 3 h. After filtration, the filter cake was washed twice with 250 mL of n-hexane, and dried at 40-50 °C to constant weight to give 214 g of INT05 as a white solid with a total yield of 69.3%.

### Example 16. Preparation of INT05

(1) In a 5 L round bottom flask were placed 150 g (0.6 mol) of INT04 and 269 g (4.8 mol, 8 eq) of solid sodium hydroxide, and 750 mL (5 Vol) of PEG-400 was added and refluxed at 120-130 °C for 15 h. The reaction was monitored via HPLC until INT04<0.5%, and then cooled to 70-80 °C. To the reaction was added 1.5 L of water (10 Vol), and well stirred. The resulting material was extracted once with 1.5 L (10 Vol) of toluene, and then extracted twice with toluene (750 mL, 5 Vol). The toluene collections were combined, and washed with saturated sodium chloride solution (2.5 L, 10 Vol), and dried with anhydrous sodium sulfate. After filtration, the filter residue was washed with toluene (250 mL, 1 Vol), and the solvent was evaporated under reduced pressure to obtain 108 g of gray crude solid.
(2) In 640 mL of dichloromethane (8 Vol) was dissolved 81 g of the crude solid, and 119 g (0.57 mol, 1.5 eq) of Boc anhydride was added, and the reaction was run at20-30 °C with stirring for 4 h, with HPLC monitoring until INT04<0.5%. After the reaction, the dichloromethane was evaporated under reduced pressure, and to the residue was added 400 mL of n-hexane for crystallization for 4 h. After filtration, the filter cake was washed twice with 80 mL of n-hexane, and dried at 40-50 °C to constant weight to give 80 g of INT05 as a white solid with a total yield of 66.8%.

### Example 17, Preparation of INT05

(1) In a 5 L round bottom flask were placed 150 g (0.6 mol) of INT04 and 144 g (3.6 mol, 6 eq) of solid potassium hydroxide, add 750 mL (5 Vol) of PEG-400 was added and refluxed at 120-130 °C for 16 h. The reaction was monitored via HPLC until INT04<0.5%, and then cooled to 70-80 °C. To the reaction was added 1.5 L of water (10 Vol), and well stirred. The resulting material was extracted once with toluene (1.5 L, 10 Vol), and then extracted twice with toluene (750 mL, 5 Vol). The toluene collections were combined, and washed with saturated sodium chloride solution (2.5 L, 10 Vol), and dried with anhydrous sodium sulfate. After filtration, the filter residue was washed with toluene (250 mL, 1 Vol), and the solvent was evaporated under reduced pressure to obtain 103 g of gray crude solid.
(2) In 820 mL of dichloromethane (10 Vol) was dissolved 82 g of crude solid, and 86 g (0.42 mol, 1.05 eq) of Boc anhydride was added, and the reaction was run at 20-30 °C with stirring for 2 h, with HPLC monitoring until INT04<0.5%. After the reaction, the dichloromethane was evaporated under reduced pressure, and to the residue was added 550 mL of n-hexane for crystallization for 3 h. After filtration, the filter cake was washed twice with 80 mL of n-hexane, and dried at 40-50 °C to constant weight to give 75.5 g of INT05 as a white solid with a total yield of 62.3%.

### Example 18. Preparation of INT05

(1) In a 100 L reactor were placed (15.9 mol) of INT04 4 kg and 5.06 kg (126.5 mol, 8 eq) of solid sodium hydroxide, and 22.3 kg (5 Vol) of PEG-400 was added and refluxed at 120-130 °C for 16 h. The reaction was monitored via HPLC until INT04<2%, and then cooled to 70-80 °C. To the reaction was added 40 L of water (10 Vol) to the reaction. , and was extracted once with toluene (40 L, 10 Vol), and then extracted twice with toluene (20 L, 5 Vol). The toluene collections were combined, and washed with saturated sodium chloride solution (40 L, 10 Vol), and dried with anhydrous sodium sulfate. After filtration, the filter residue was washed with toluene (4 L, 1 Vol), and the solvent was evaporated under reduced pressure to obtain a gray crude solid.
(2) In 40 L of dichloromethane (10 Vol) was dissolved the crude solid, and 3.56 kg (16.3 mol, 1.05 eq) of Boc anhydride was added, and the reaction was run at 20-30 °C with stirring for 2 h, with HPLC monitoring until INT04<0.5%. After the reaction, the dichloromethane was evaporated under reduced pressure, and to the residue was added 15.1 kg of n-hexane for crystallization for 5 h. After filtration, the filter cake was washed twice with 2.1 kg n-hexane, and dried at 40-50 °C to constant weight to give 3.5 kg of INT05 as a white solid with a total yield of 71.1%.

### Example 19, Preparation of INT06

To 3.8 mL (40.8 mmol, 1.8 eq) of acetic anhydride, which was cooled to 0-10 °C, was slowly added dropwise 2.8 mL (63.5 mmol, 2.8 eq) of fuming nitric acid, and then the resulting mixture was stirred for 30 min. A solid of INT05 (7 g, 22.7 mmol) was dissolved in 70 mL of dichloromethane with cooling to 0-10 °C, and the prepared nitrating reagent was added dropwise. The reaction was kept at 0-10 °C and run for 6 h. Samples were taken at 0, 0.5, 1.5, 3, and 6 hours after the dropwise addition, and the reaction was monitored via HPLC to check at different time points the conditions of the raw materials, products and impurities, the results of which are shown in the table below. The raw materials were completed consumed after the dropwise addition, at the same time the by-products began to form, and the by-products reached 15% at 0.5 h of the reaction, INT06 dropped to 33% at 3 h of the reaction, and INT06 dropped to about 18% at 6 h of the reaction.

| Time | INT05 | INT06 | Impurities |
|---|---|---|---|
| 0 h | N.D. | 75.30% | 1.35% |
| 0.5 h | N.D. | 63.13% | 14.48% |
| 1.5 h | N.D. | 47.82% | 32.63% |
| 3 h | N.D. | 32.63% | 50.36% |
| 6 h | N.D. | 17.91% | 63.77% |

### Example 20. Preparation of INT06

To 3.42 mL (32.6 mmol, 1.62 eq) of acetic anhydride, which was cooled to 0-10 °C, was slowly added dropwise 2.52 mL (57.5 mmol, 2.52 eq) of fuming nitric acid, and then the resulting mixture was stirred for 30 min. A solid of INT05 (7 g, 22.7 mmol) was dissolved in 70 mL of dichloromethane with cooling to 0-10 °C, and the prepared nitrating reagent was added dropwise. The reaction was kept at 0-10 °C and run for 6 h. Samples were taken at 0, 0.5, 1.5, 3 and 6 hours after the dropwise addition, and the reaction was monitored via HPLC to check at different time points the conditions of the raw materials, products and impurities, the results of which are shown in the table below. The raw materials were completed consumed after the addition, at the same time the by-products began to form, and the by-products reached 6% at 0.5 h of the reaction, INT06 dropped to 56% at 3 h of the reaction, and INT06 dropped to about 43% at 6 h of the reaction.

| Time | INT05 | INT06 | Impurities |
|---|---|---|---|
| 0 h | N.D. | 77.22% | 0.89% |
| 0.5 h | N.D. | 73.17% | 6.08% |
| 1.5 h | N.D. | 65.22% | 14.40% |
| 3 h | N.D. | 55.82% | 23.65% |
| 6 h | N.D. | 43.15% | 36.98% |

### Example 21. Preparation of INT06

To 3.04 mL (36.7 mmol, 1.44 eq) of acetic anhydride, which was cooled to 0-10 °C, was slowly added dropwise 2.24 mL (50.8 mmol, 2.24 eq) of fuming nitric acid, and then the resulting mixture was stirred for 30 min. A solid of INT05 (7 g, 22.7 mmol) was dissolved in 70 mL of dichloromethane with cooling to 0-10 °C, and the prepared nitrating reagent was added dropwise. The reaction was kept at 0-10 °C and run for 6 h. Samples were taken at 0, 0.5, 1.5, 3, and 6 hours after the dropwise addition, and the reaction was monitored via HPLC to check at different time points the conditions of the raw materials, products and impurities, the results of which are shown in the table below. The raw materials were completed consumed 0.5 h after the addition, and at the same time the by-products reached 3.5%, and the by-product was about 12% at 3 h of the reaction.

| Time | INT05 | INT06 | Impurities |
|---|---|---|---|
| 0 h | 0.68% | 76.55% | 0.32% |
| 0.5 h | N.D. | 75.24% | 3.50% |
| 1.5 h | N.D. | 73.88% | 6.30% |
| 3 h | N.D. | 67.42% | 11.73% |
| 6 h | N.D. | 58.22% | 20.77% |

### Example 22. Preparation of INT06

To 2.66 mL (28.6 mmol, 1.26 eq) of acetic anhydride, which was cooled to 0-10 °C, was slowly added dropwise 1.96 mL (44.5 mmol, 1.96 eq) of fuming nitric acid, and then the resulting mixture was stirred for 30 min. A solid of INT05 (7 g, 22.7 mmol) was dissolved in 70 mL of dichloromethane with cooling to 0-10 °C, and the prepared nitrating reagent was added dropwise. The reaction was kept at 0-10 °C and run for 6 h. Samples were taken at 0, 0.5, 1.5, 3, and 6 hours after the dropwise addition, and the reaction was monitored via HPLC to check at different time points the conditions of the raw materials, products and impurities, the results of which are shown in the table below. The raw material remained 0.37% at 0.5 h after the dropwise addition, which met the requirements of central control. The raw material reacted completely within 3 h of the reaction, and the by-product was 1.84%.

| Time | INT05 | INT06 | Impurities |
|---|---|---|---|
| 0 h | 3.42% | 71.20% | N.D. |
| 0.5 h | 0.37% | 77.13% | 0.94% |
| 1.5 h | 0.23% | 77.55% | 1.37% |
| 3 h | N.D. | 77.61% | 1.84% |
| 6 h | N.D. | 76.33% | 2.79% |

### Example 23. Preparation of INT06

To 2.28 mL (24.5 mmol, 1.08 eq) of acetic anhydride, which was cooled to 0-10 °C, was slowly added dropwise 1.68 mL (38.1 mmol, 1.68 eq) of fuming nitric acid, and then the resulting mixture was stirred for 30 min. A solid of INT05 (7 g, 22.7 mmol) was dissolved in 70 mL of dichloromethane with cooling to 0-10 °C, and the prepared nitrating reagent was added dropwise. The reaction was kept at 0-10 °C and run for 6 h. Samples were taken at 0, 0.5, 1.5, 3 and 6 hours after the dropwise addition, and the reaction was monitored via HPLC to check at different time points the conditions of the raw materials, products and impurities, the results of which are shown in the table below. The raw material remained 0.49% after the dropwise addition, and the by-product level was 0.78%, and after 6 h of the reaction the raw material completely consumed, and INT06 was about 79%.

| Time | INT05 | INT06 | Impurities |
|---|---|---|---|
| 0 h | 10.22% | 57.33% | N.D. |
| 0.5 h | 0.59% | 77.56% | 0.51% |
| 1.5 h | 0.72% | 78.06% | 0.69% |
| 3 h | 0.49% | 77.50% | 0.67% |
| 6 h | N.D. | 78.64% | 0.74% |

### Example 24. Preparation of INT06

(1) To 114 mL (1.2 mol, 1.8 eq) of acetic anhydride, which was cooled to 0-10 °C, was slowly added dropwise 84 mL (1.9 mol, 2.8 eq) of fuming nitric acid, and then the resulting mixture was stirred for 30 min. A solid of 210 g (0.68 mol) of INT05 was dissolved in 2.1 L (10 Vol) of dichloromethane with cooling to 0-10 °C, and the prepared nitrating reagent was added dropwise. The reaction was kept at 0-10 °C and run for 30 min. The reaction was monitored via HPLC until INT05 < 0.5%, and then 2.1 L of ice water (10 Vol) was added to quench the reaction. After stirring, the aqueous layer was separated, and the organic layer was washed with 2.1 L (10 Vol) of 1N sodium bicarbonate solution and 2.1 L (10 Vol) of saturated sodium chloride solution, and further dried with anhydrous sodium sulfate. After filtration, the filter residue was washed twice with 210 mL of dichloromethane. The dichloromethane phased were combined and concentrated to evaporate the solvent under reduced pressure at 30-35°C to give a yellow oily liquid.
(2) To the yellow oily liquid was added 480 mL of ethanol, which was stirred until complete dissolution, and 30 mL of water was added. The reaction was controlled with the temperature at 20-30 °C and stirred until forming of crystals. The resulting suspension was added with remaining 420 mL of water, kept at 20-30 °C, and stirred for 2 h. After suction filtration, the filter cake was sucked dry, and then dried in vacuum at 40-50 °C to constant weight to give 200 g of INT06 as a white solid with a yield of 83%.

### Example 25. Preparation of INT06

(1) To 14.6 mL (157 mmol, 1.62 eq) of acetic anhydride, which was cooled to 0-10 °C, was slowly added dropwise 10.8 mL (244 mmol, 2.52 eq) of fuming nitric acid, and then the resulting mixture was stirred for 30 min. A solid of 30 g (97 mmol) of INT05 was dissolved in 300 mL (10 Vol) of dichloromethane with cooling to 0-10 °C, and the prepared nitrating reagent was added dropwise. The reaction was kept at 0-10 °C and run for 30 min. The reaction was monitored via HPLC until INT05 < 0.5%, and then 300 mL of ice water (10 Vol) was added to quench the reaction. After stirring, the aqueous layer was separated, and the organic layer was washed with 300 mL (10 Vol) of 1N sodium bicarbonate solution and 300 mL (10 Vol) of saturated sodium chloride solution, and further dried with anhydrous sodium sulfate. After filtration, the filter residue was washed twice with 30 mL of dichloromethane. The dichloromethane phased were combined and concentrated to evaporate the solvent under reduced pressure at 30-35°C to give a yellow oily liquid.
(2) To the yellow oily liquid was added 127 mL of ethanol, which was stirred until complete dissolution, and 30 mL of water was added. The reaction was controlled with the temperature at 20-30 °C and stirred until forming of crystals. The resulting suspension was added with remaining 60 mL of water, kept at 20-30 °C, and stirred for 2 h. After suction filtration, the filter cake was sucked dry, and then dried in vacuum at 40-50 °C to constant weight to give 27.4 g of INT06 as a white solid with a yield of 80%.

### Example 26. Preparation of INT06

(1) To 13.0 mL (0.14 mol, 1.44 eq) of acetic anhydride, which was cooled to 0-10 °C, was slowly added dropwise 9.6 mL (0.22 mol, 2.24 eq) of fuming nitric acid, and then the resulting mixture was stirred for 30 min. A solid of 30 g (97 mmol) of INT05 was dissolved in 300 mL (10 Vol) of dichloromethane with cooling to 0-10 °C, and the prepared nitrating reagent was added dropwise. The reaction was kept at 0-10 °C and run for 30 min. The reaction was monitored via HPLC until INT05 < 0.5%, and then 300 mL of ice water (10 Vol) was added to quench the reaction. After stirring, the aqueous layer was separated, and the organic layer was washed with 300 mL (10 Vol) of 1N sodium bicarbonate solution and 300 mL (10 Vol) of saturated sodium chloride solution, and further dried with anhydrous sodium sulfate. After filtration, the filter residue was washed twice with 30 mL of dichloromethane. The dichloromethane phased were combined and concentrated to evaporate the solvent under reduced pressure at 30-35°C to give a yellow oily liquid.
(2) To the yellow oily liquid was added 127 mL of ethanol, which was stirred until complete dissolution, and 30 mL of water was added. The reaction was controlled with the temperature at 20-30 °C and stirred until forming of crystals. The resulting suspension was added with remaining 60 mL of water, kept at 20-30 °C, and stirred for 2 h. After suction filtration, the filter cake was sucked dry, and then dried in vacuum at 40-50 °C to constant weight to give 26.8 g of INT06 as a white solid with a yield of 78%.

### Example 27. Preparation of INT06

(1) To 15.2 mL (0.16 mol, 1.26 eq) of acetic anhydride, which was cooled to 0-10 °C, was slowly added dropwise 11.2 mL (0.25 mol, 1.96 eq) of fuming nitric acid, and then the resulting mixture was stirred for 30 min. A solid of 40 g (0.13 mol) of INT05 was dissolved in 400 mL (10 Vol) of dichloromethane with cooling to 0-10 °C, and the prepared nitrating reagent was added dropwise. The reaction was kept at 0-10 °C and run for 6 h. The reaction was monitored via HPLC until INT05 < 0.5%, and then 400 mL of ice water (10 Vol) was added to quench the reaction. After stirring, the aqueous layer was separated, and the organic layer was washed with 400 mL (10 Vol) of 1N sodium bicarbonate solution and 400 mL (10 Vol) of saturated sodium chloride solution, and further dried with anhydrous sodium sulfate. After filtration, the filter residue was washed twice with 40 mL of dichloromethane. The dichloromethane phased were combined and concentrated to evaporate the solvent under reduced pressure at 30-35°C to give a yellow oily liquid.
(2) To the yellow oily liquid was added 157 mL of ethanol, which was stirred until complete dissolution, and 40 mL of water was added. The reaction was controlled with the temperature at 20-30 °C and stirred until forming of crystals. The resulting suspension was added with remaining 80 mL of water, kept at 20-30 °C, and stirred for 2 h. After suction filtration, the filter cake was sucked dry, and then dried in vacuum at 40-50 °C to constant weight to give 40.7 g of INT06 as a white solid with a yield of 89%.

### Example 28. Preparation of INT06

(1) To 13.0 mL (0.14 mol, 1.08 eq) of acetic anhydride, which was cooled to 0-10 °C, was slowly added dropwise 9.6 mL (0.22 mol, 1.68 eq) of fuming nitric acid, and then the resulting mixture was stirred for 30 min. A solid of 40 g (0.13 mol) of INT05 was dissolved in 400 mL (10 Vol) of dichloromethane with cooling to 0-10 °C, and the prepared nitrating reagent was added dropwise. The reaction was kept at 0-10 °C and run for 6 h. The reaction was monitored via HPLC until INT05 < 0.5%, and then 400 mL of ice water (10 Vol) was added to quench the reaction. After stirring, the aqueous layer was separated, and the organic layer was washed with 400 mL (10 Vol) of 1N sodium bicarbonate solution and 400 mL (10 Vol) of saturated sodium chloride solution, and further dried with anhydrous sodium sulfate. After filtration, the filter residue was washed twice with 40 mL of dichloromethane. The dichloromethane phased were combined and concentrated to evaporate the solvent under reduced pressure at 30-35°C to give a yellow oily liquid.
(2) To the yellow oily liquid was added 157 mL of ethanol, which was stirred until complete dissolution, and 40 mL of water was added. The reaction was controlled with the temperature at 20-30 °C and stirred until forming of crystals. The resulting suspension was added with remaining 80 mL of water, kept at 20-30 °C, and stirred for 2 h. After suction filtration, the filter cake was sucked dry, and then dried in vacuum at 40-50 °C to constant weight to give 38.9 g of INT06 as a white solid with a yield of 85%.

### Example 29. Preparation of INT06

(1) To 1.45 kg (14.3 mol, 1.26 eq) of acetic anhydride, which was cooled to 0-10 °C, was slowly added dropwise 1.47 kg (22.17 mol, 1.96 eq) of fuming nitric acid, and then the resulting mixture was stirred for 30 min. A solid of 3.5 kg (11.3 mol) of INT05 was dissolved in 35 L (10 Vol) of dichloromethane with cooling to 0-10 °C, and the prepared nitrating reagent was added dropwise. The reaction was kept at 0-10 °C and run for 0.5 h. The reaction was monitored via HPLC until INT05 < 0.5%, and then 5 L of ice water (10 Vol) was added to quench the reaction. After stirring, the aqueous layer was separated, and the organic layer was washed with 35 L (10 Vol) of 1N sodium bicarbonate solution and 35 L (10 Vol) of saturated sodium chloride solution, and further dried with anhydrous sodium sulfate. After filtration, the filter residue was washed twice with 35 L of dichloromethane. The dichloromethane phased were combined and concentrated to evaporate the solvent under reduced pressure at 30-35°C to give a yellow oily liquid.
(2) To the yellow oily liquid was added 13.65 kg of ethanol, which was stirred until complete dissolution, and 3.5 L of water was added. The reaction was controlled with the temperature at 20-30 °C and stirred until forming of crystals. The resulting suspension was added with remaining 7 L of water, kept at 20-30 °C, and stirred for 2 h. After suction filtration, the filter cake was sucked dry, and then dried in vacuum at 40-50 °C to constant weight to give 3.8 kg of INT06 as a white solid with a yield of 94%.

### Example 30. Preparation of MN08

(1) HCl gas was introduced into ethyl acetate to form an HCl/ethyl acetate solution with a concentration of 4.37 M. In 1 L of ethyl acetate was dissolved 200 g (0.56 mol) of INT06, and 646 mL of HCl/ethyl acetate solution (HCl 2.8 mol, 5 eq) was added. The reaction was controlled with the temperature at 20-30 °C and run for 16 h. The reaction was monitored via HPLC, and terminated when INT06 < 0.5%. The resulting mixture was filtered with suction, and the filter cake was washed with 400 mL of ethyl acetate, and sucked to dry to give crude MN08.
(2) The crude MN08 was dissolved completely in 500 mL of ethanol and filtered with suction, and the filter cake was washed with 80 mL of ethanol. The combined ethanol was distilled under reduced pressure at 40-50 °C to a residue with 100 mL of ethanol remaining, the temperature was lowered to 20-30 °C, and 4 L of ethyl acetate was added with stirring for 2 h. The resulting mixture was filtered with suction, and the filter cake was washed with 200 mL of ethyl acetate, and dried under reduced pressure at 40-50 °C to constant weight, to give 128 g of MN08 as a white solid with a yield of 78%.

### Example 31. Preparation of MN08

(1) HCl gas was introduced into ethyl acetate to form an HCl/ethyl acetate solution with a concentration of 3.7 M. In 465 mL of ethyl acetate was dissolved 93 g (0.26 mol) of INT06, and 700 mL of HCl/ethyl acetate solution (2.6 mol HCl, 10 eq) was added. The reaction was controlled with the temperature at 20-30 °C and run for 16 h. The reaction was monitored via HPLC, and terminated when INT06 < 0.5%. The resulting mixture was filtered with suction, and the filter cake was washed with 190 mL of ethyl acetate, and sucked to dry to give crude MN08.
(2) The crude MN08 was dissolved completely in 280 mL of ethanol and filtered with suction, and the filter cake was washed with 80 mL of ethanol. The combined ethanol was distilled under reduced pressure at 40-50 °C to a residue with 50 mL of ethanol remaining, the temperature was lowered to 20-30 °C, and 1.4 L of ethyl acetate was added with stirring for 2 h. The resulting mixture was filtered with suction, and the filter cake was washed with 100 mL of ethyl acetate, and dried under reduced pressure at 40-50 °C to constant weight, to give 58 g of MN08 as a white solid with a yield of 76%.

### Example 32. Preparation of MN08

(1) HCl gas was introduced into ethyl acetate to form an HCl/ethyl acetate solution with a concentration of 3.7 M. In 590 mL of ethyl acetate was dissolved 118 g (0.33 mol) of INT06, and 446 mL of HCl/ethyl acetate solution (1.65 mol HCl, 5 eq) was added. The reaction was controlled with the temperature at 20-30 °C and run for 16 h. The reaction was monitored via HPLC, and terminated when INT06 < 0.5%. The resulting mixture was filtered with suction, and the filter cake was washed with 259 mL of ethyl acetate, and sucked to dry to give crude MN08.
(2) The crude MN08 was dissolved completely in 360 mL of ethanol and filtered with suction, and the filter cake was washed with 120 mL of ethanol. The combined ethanol was distilled under reduced pressure at 40-50 °C to a residue with 100 mL of ethanol remaining, the temperature was lowered to 20-30 °C, and 2.4 L of ethyl acetate was added with stirring for 2 h. The resulting mixture was filtered with suction, and the filter cake was washed with 200 mL of ethyl acetate, and dried under reduced pressure at 40-50 °C to constant weight, to give 70 g of MN08 as a white solid with a yield of 83%.

### Example 33. Preparation of MN08

(1) HCl gas was introduced into ethyl acetate to form an HCl/ethyl acetate solution with a concentration of 4.37 M. In 22.5 L of ethyl acetate was dissolved 4.5 kg (12.7 mol) of INT06, and 14.5 L of HCl/ethyl acetate solution (63.6 mol HCl, 5 eq) was added. The reaction was controlled with the temperature at 20-30 °C and run for 16 h. The reaction was monitored via HPLC, and terminated when INT06 < 0.5%. The resulting mixture was filtered with suction, and the filter cake was washed with 9 L of ethyl acetate, and sucked to dry to give crude MN08.
(2) The crude MN08 was dissolved completely in 12 L of ethanol and filtered with suction, and the filter cake was washed with 1.5 L of ethanol. The combined ethanol was distilled under reduced pressure at 40-50 °C to a residue with 2 L of ethanol remaining, the temperature was lowered to 20-30 °C, and 90 L of ethyl acetate was added with stirring for 2 h. The resulting mixture was filtered with suction, and the filter cake was washed with 4 L ethyl acetate, and dried under reduced pressure at 40-50 °C to constant weight, to give 2.4 kg of MN08 as a white solid with a yield of 65%.

Some embodiments and examples of the present invention are provided herein for the purpose of illustration, and they are not used to limit the scope of the invention.

## Claims

1. A process for manufacture of amantadine nitrate derivatives, comprising following steps: (1) synthesis of adamantanol from adamantane; (2) carboxylation of adamantanol; (3) acetylation of adamantanoic acid; (4) reduction; (5) hydrolysis of amido adamantanol and Boc protection of amino group; (6) crystallization of Boc protected amantadinol; (7) nitrate esterification of Boc protected amantadinol; (8) refining of the product of nitrate esterification; (9) Boc deprotection and salt formation; and (10) refining of amantadine nitrate hydrochloride: 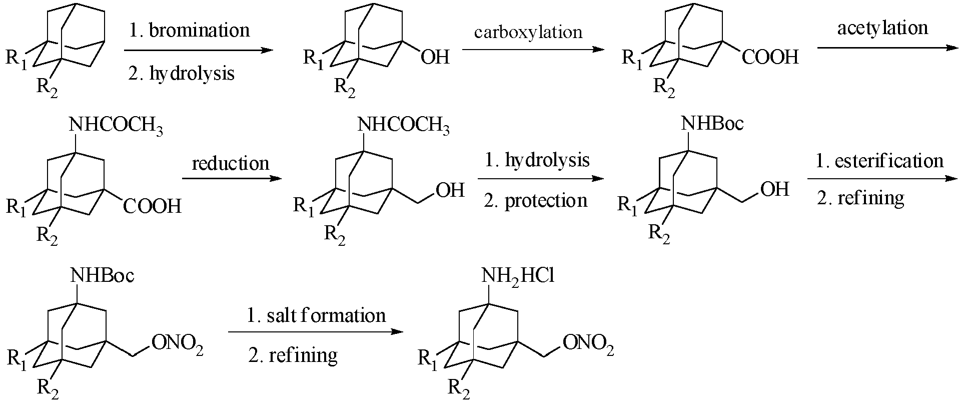 wherein, the adamantane has the structure of: 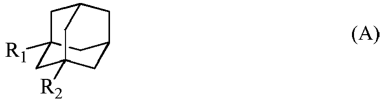 wherein, R₁ and R₂ are each independently hydrogen, straight-chain or branched-chain alkyl, substituted or unsubstituted aryl or heteroaryl;
wherein, the steps further comprise:
(1) synthesis of adamantanol: treating a substituted or unsubstituted adamantane to be brominated with liquid bromine and hydrolyzed with sodium oxalate to form an adamantanol;
(2) carboxylation of adamantanol: treating the adamantanol via Koch-Haaf reaction, and condensation with formic acid to obtain an adamantanoic acid;
(3) acetylation of adamantanoic acid: treating the adamantanoic acid via condensation with acetonitrile via Ritter reaction to obtain an amido adamantanic acid;
(4) reduction: treating the amido adamantanic acid via reduction with sodium borohydride to obtain an amido adamantanol;
(5) hydrolysis of amido adamantanol and Boc protection of amino group: treating the amido adamantanol, after an acyl group thereof being removed with strong alkali hydrolysis, via condensation with Boc anhydride;
(6) crystallization of Boc protected amantadinol: treating the product of Boc condensation via crystallization with mashing in n-hexane;
(7) nitrate esterification of Boc protected amantadinol: reacting the Boc protected amantadinol with a nitrating agent at a temperature of -10°C to +10°C to obtain a product of nitrate esterification, wherein the nitrating agent is a reagent with a mixture of fuming nitric acid and acetic anhydride;
(8) refining of the product of nitrate esterification: dissolving the product of nitrate esterification in alcohol, and adding water for crystallization with mashing;
(9) Boc deprotection and salt formation: removing Boc protecting group of the product obtained in step (8) via hydrolysis with HCl to obtain amantadine nitrate hydrochloride; and
(10) refining of amantadine nitrate hydrochloride: dissolving the amantadine nitrate hydrochloride in ethanol, resulting solution being filtered, concentrated and evaporated to remove partially the ethanol, adding ethyl acetate for crystallization with mashing, to obtain, after centrifugation, an amantadine nitrate hydrochloride;
wherein, in step (5), the reaction solvent for hydrolysis is polyethylene glycol 400; and in step (7), the molar ratio of the reaction feed is raw material : acetic anhydride : fuming nitric acid = 1 : 1.08-1.8 : 1.68-2.8; and in step (9), the molar ratio of reaction feed is raw material : HCl = 1 : 5-10, the reaction temperature is from 20°C to 30°C and the reaction time is from 16 to 18 hours.

2. The process according to claim 1, wherein step (1) further comprises:
(1a) Bromination: reacting a substituted or unsubstituted adamantane with liquid bromine in reflux for 4-6 h to obtain bromoadamantane; and
(1b) Hydrolysis: adding sodium oxalate and water, the reaction system being refluxed at 75 °C via hydrolization to obtain a substituted or unsubstituted adamantanol.

3. The process according to claim 1, wherein step (2) further comprises: the product from step (1) being dissolved in formic acid, and slowly added dropwise to concentrated sulfuric acid with cooling to 0-10 °C, and then the reaction being maintained at 0-10 °C for 3-6 h; the reaction solution being poured into ice water and stirred for 16-18 h; after suction filtration, the solid filter cake being washed with water, dissolved with 0.27X sodium hydroxide solution, and filtered with suction; the filtrate being acidified with 5-10% hydrochloric acid to pH=1-2 and filtered, the filter cake being washed with water until the filtrate being neutral, and dried at 40-50 °C to obtain adamantanoic acid.

4. The process according to claim 1, wherein step (3) further comprises: cooling concentrated sulfuric acid to 0-10 °C, and adding adamantanoic acid to be dissolved with stirring, adding slowly dropwise nitric acid, and then adding dropwise acetonitrile, reaction being maintained at 0-10 °C for 2-3 h; reaction solution being poured into ice water with stirring for 16-18 h, and filtered with suction, and filter cake being washed with water and dried at 40-50 °C to obtain amido adamantanic acid.

5. The process according to claim 1, wherein step (4) further comprises: adding the product of step (3) to tetrahydrofuran, cooled to 0-10 °C, and adding triethylamine in batches, then adding dropwise ethyl chloroformate, and reaction being run at room temperature for 4-6 h; filtering resulting reaction mixture, and washing filter cake with tetrahydrofuran, combing tetrahydrofuran phases and cooling to 0-10 °C, adding sodium borohydride in batches, then adding 0.8-1X water dropwise, and reaction being run for 2-3 h; adding 5X water, filtering reaction mixture, filtrate being spin-dried, then extracted with ethyl acetate, and combined ethyl acetate phases being dried over sodium sulfate, filtered, and spin dried, and residue being added with 1.5-2.5X ethyl acetate, stirred well, the same amount of petroleum ether being added, and stirred for 12-16 h, filtered and filter cake dried at 40-50 °C to obtain an amido adamantanol.

6. The process according to claim 1, wherein step (5) comprises following reactions:
(5a) Hydrolysis: adding amido adamantanol and a strong base sequentially to polyethylene glycol with a 5-10 times volume, reacted via high temperature hydrolysis at 100-180 °C for 10-18 h, and reaction system being cooled, diluted with purified water and stirred evenly, and water layer being extracted with toluene, dried over sodium sulfate and filtered, and the filtrate being concentrated; and
(5b) Condensation: dissolving the product obtained in reaction (5a) in dichloromethane, adding Boc anhydride, then running reaction via condensation at 20-30 °C for 1-5 h, and resulting mixture being concentrated to remove dichloromethane.

7. The process according to claim 1, wherein step (6) further comprises: adding n-Hexane to resulting residual oily liquid of step (5), reacted via crystallization for 2-3 h, and the resulting mixture being centrifuged, washed with n-hexane, and then dried at 40-50 °C for 16-18 h to obtain Boc-protected amantadinol.

8. The process according to claim 1, wherein step (7) further comprises: mixing fuming nitric acid with acetic anhydride at a temperature of -10 °C to 10 °C to give a nitrating reagent; dissolving resulting solid product from step (6) in dichloromethane with temperature controlled at -10°C to 10°C, adding dropwise the nitrating reagent to be reacted for 15 min to 6 h, then quenching reaction upon its completion with ice water and water layer being separated, and organic layer being washed respectively with 10 times volume (10 Vol) of a solution of saturated sodium bicarbonate and a solution of saturated sodium chloride, dried over sodium sulfate, filtered, and concentrated to remove dichloromethane to obtain a crude product of Boc-protected amantadine nitrate.

9. The process according to claim 1, wherein step (8) further comprises: dissolving the crude product of step (7) in alcohol, adding water for crystallization with mashing for 2-3 h, then the resulting mixture being filtered, and resulting solid being dried at 40-50 °C to obtain a refined product.

10. The process according to claim 1, step (9) further comprises: dissolving the refined product of step (8) in ethyl acetate with temperature controlled at 10-30 °C, adding HCl/ethyl acetate, running reaction for 16-18 h, and then resulting mixture being filtered with suction, and dried to obtain an amantadine nitrate hydrochloride.

11. The process according to claim 1, step (10) further comprises: adding product of step (9) in ethanol, treating via filtration with filtrate being concentrated, and adding ethyl acetate for crystallization with beating, resulting mixture being dried to constant weight to obtain a refined amantadine nitrate hydrochloride.

12. The process according to claim 2, wherein, in reaction of (1a) bromination of step (1), molar ratio of reaction feed is raw material : bromine = 1 : 2-6, reaction temperature is 60-90 °C, and reaction time is 4 to 6 h; wherein, in reaction of (1b) hydrolysis of step (1), molar ratio of reaction feed is raw material : sodium oxalate = 1 : 2.5-6.0, amount of water used is 23-35X, and reaction time is 2 to 3 h; or wherein, in step (1) post-treatment process comprises: adding 3-5X sodium sulfite solution to reaction solution, stirring at room temperature for 16-18 h and then filtered, and filter cake being washed with water and then dissolved with 3-8X organic solvent, dried over anhydrous sodium sulfate, and filtered, and filter cake being washed with 2-5X organic solvent, and spin-dried under reduced pressure to obtain adamantanol as a solid.

13. The process according to claim 3, wherein, in step (2), molar ratio of reaction feed is raw material : formic acid : sulfuric acid = 1 : 6-10 : 11-16.

14. The process according to claim 1, wherein, in step (3), molar ratio of reaction feed is raw material : nitric acid: sulfuric acid: acetonitrile = 1 : 1.6-2.5 : 9-15 : 1.1-1.5; or wherein, in step (4), molar ratio of reaction feed is raw material : triethylamine : ethyl chloroformate : sodium borohydride = 1 : 1.2-1.5 : 1.2- 1.5 : 1.5-2.5.

15. The process according to claim 6, wherein, in the hydrolysis of step (5), the strong base is lithium hydroxide, sodium hydroxide or potassium hydroxide, and molar ratio of reaction feed is raw material : base = 1 : 5-10; wherein, reaction temperature is 100-180 °C, reaction time is 15-16 h; or wherein, in the condensation of step (5), amount of dichloromethane is 5-10 times in volume of raw material, and molar ratio of reaction feed is raw material : Boc anhydride = 1 : 1.05-1.5, and reaction time is 1-5 h.

16. The process according to claim 7, wherein in step (6), the amount of n-hexane used for crystallization is 5-9 times in volume of raw material.

17. The process according to claim 8, wherein in step (7), molar ratio of reactants is raw material : acetic anhydride : fuming nitric acid = 1 : 1.08-1.8 : 1.68-2.8; or wherein the nitrating reagent is prepared at -10-10 °C and stirred for 0.5-1 h; and wherein reaction temperature is 0-10 °C, and reaction time is 15 min to 6 h, or 30 min to 3 h.

18. The process according to claim 9, wherein in the step (8), the alcohol used for refining is methanol or ethanol, and weight ratio of reaction feed is raw material : alcohol : water = 1: 2-5 : 3-8; wherein, after crude product of step (7) is completely dissolved in alcohol, a portion of the water was added, and stirred until precipitation of a white solid, then further stirred for 0.5-1 h, and remaining portion of the water was added with beating for 1-2 h; and wherein temperature for crystallization is controlled at 20-30 °C.

19. The process according to claim 10, wherein, in step (9), concentration of HCl in HCl/ethyl acetate is in the range of 2.0-4.37 M, molar ratio of reaction feed is raw material : HCl = 1 : 5- 10, reaction temperature is 20-30 °C, and reaction time is 16-18 h; and wherein, refined product of step (8) is first dissolved in 3-6 times the weight of ethyl acetate, and then HCl/ethyl acetate is added for reaction.

20. The process according to claim 11, wherein, step (10) further comprising: dissolving the product of step (9) in alcohol, and concentrated under reduced pressure to remain 0.5-1 times of ethanol in the system, then adding ethyl acetate with mashing for 2-3 h, and wherein weight ratio of reaction feed is raw material : ethanol : ethyl acetate = 1 : 2.5-3 : 15-25, temperature of concentration with ethanol is 40-50 °C, and temperature for drying is 45-50 °C.

## Patentansprüche

1. Verfahren zur Herstellung von Amantadinnitratderivaten, umfassend die Schritte: (1) Synthese von Adamantanol aus Adamantan; (2) Carboxylierung von Adamantanol; (3) Acetylierung von Adamantansäure; (4) Reduktion; (5) Hydrolyse von Amidoadamantanol und Schützen der Aminogruppe mittels tert-Butyloxycarbonyl (Boc); (6) Kristallisation von Boc-geschütztem Amantadinol; (7) Nitratveresterung von Boc-geschütztem Amantadinol; (8) Aufreinigung des Produkts der Nitratveresterung; (9) Boc-Entschützen und Salzbildung; und (10) Aufreinigen von Amantadinnitrat-Hydrochlorid. 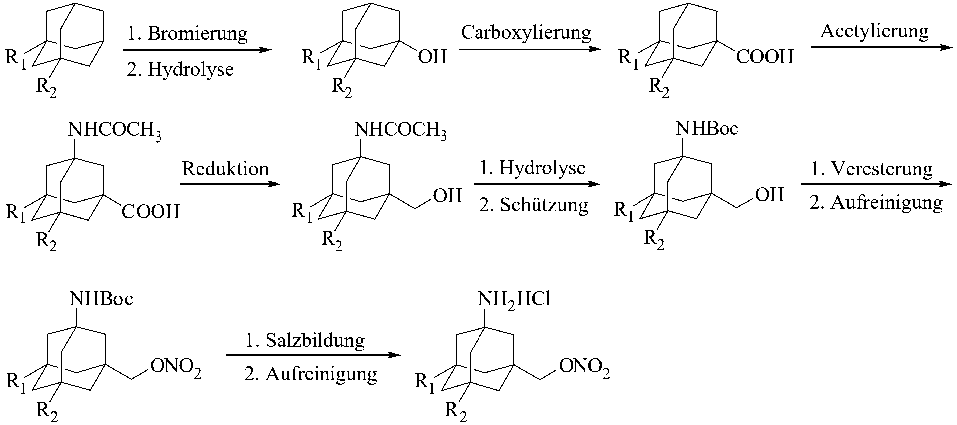 wobei das Adamantan die Struktur (A) hat: 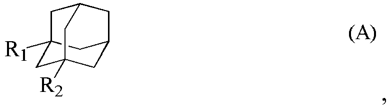
in der R₁ und R₂ jeweils unabhängig voneinander Wasserstoff, lineares oder verzweigtes Alkyl, substituiertes oder unsubstituiertes Aryl oder Heteroaryl sind;
wobei die Schritte weiterhin umfassen:
(1) Synthese von Adamantanol: Bromieren von substituiertem oder unsubstituiertem Adamantan mit flüssigem Brom und Hydrolysieren mit Natriumoxalat zur Bildung von Adamantanol;
(2) Carboxylierung von Adamantanol: Umwandeln des Adamantanols mittels Koch-Haaf-Reaktion und Kondensation mit Ameisensäure, um Adamantansäure zu erhalten.;
(3) Acetylierung von Adamantansäure: Kondensation der Adamantansäure mit Acetonitril mittels Ritter-Reaktion, um Amidoadamantansäure zu erhalten;
(4) Reduktion: Reduktion der Amidoadamantansäure mit Natriumborhydrid, um Amidoadamantanol zu erhalten;
(5) Hydrolyse von Amidoadamantanol und Schützen der Aminogruppe mittels tert-Butyloxycarbonyl (Boc): Kondensation des Amidoadamantanols, nach Entfernen der Acylgruppe durch alkalische Hydrolyse, mit Boc-Anhydrid;
(6) Kristallisation von Boc-geschütztem Amantadinol: Kristallisieren des Produkts der Boc-Kondensation in n-Hexan und Zerkleinern;
(7) Nitratveresterung von Boc-geschütztem Amantadinol: Behandeln des Boc-geschützten Amantadinols mit einem Nitrierungsmittel bei einer Temperatur von -10°C bis +10 °C, um ein Produkt der Nitratveresterung zu erhalten, wobei das Nitrierungsmittel ein Gemisch aus rauchender Salpetersäure und Essigsäureanhydrid ist;
(8) Aufreinigung des Produkts der Nitratveresterung: Lösen des Produkts der Nitratveresterung in Alkohol und Zugeben von Wasser zur Kristallisation und Zerkleinern;
(9) Boc-Entschützen und Salzbildung: Entfernen der Boc-Schutzgruppe des in Schritt (8) erhaltenen Produkts durch Hydrolyse mit HCl, um Amantadinnitrat-Hydrochlorid zu erhalten; und
(10) Aufreinigen von Amantadinnitrat-Hydrochlorid: Lösen des Amantadinnitrat-Hydrochlorids in Ethanol; Filtrieren der resultierenden Lösung; Konzentrieren und Verdampfen, um das Ethanol teilweise zu entfernen; Zugeben von Ethylacetat zur Kristallisation und Zerkleinern, um nach zentrifugieren das Amantadinnitrat-Hydrochlorid zu erhalten;
wobei in Schritt (5) das Reaktionslösungsmittel für die Hydrolyse Polyethylenglykol 400 ist; in Schritt (7) das Molverhältnis der eingesetzten Reaktionskomponenten wie folgt ist Rohmaterial : Essigsäureanhydrid : rauchende Salpetersäure = 1 : 1,08-1,8 : 1,68-2,8; und in Schritt (9) das Molverhältnis der eingesetzten Reaktionskomponenten wie folgt ist: Rohmaterial: HCl = 1 : 5-10, wobei die Reaktionstemperatur zwischen 20°C und 30°C liegt und die Reaktionszeit zwischen 16 und 18 Stunden beträgt.

2. Verfahren nach Anspruch 1, wobei Schritt (1) weiterhin umfasst:
(1a) Bromierung: Behandeln eines substituierten oder unsubstituierten Adamantans mit flüssigem Brom unter Rückfluss für 4 bis 6 Stunden, um Bromadamantan zu erhalten; und
(1b) Hydrolyse: Zugeben von Natriumoxalat und Wasser, wobei das Reaktionssystem bei 75°C unter Rückfluss hydrolysiert wird, um ein substituiertes oder unsubstituiertes Adamantanol zu erhalten.

3. Verfahren nach Anspruch 1, wobei Schritt (2) weiterhin umfasst: Lösen des Produkts aus Schritt (1) in Ameisensäure und langsame tropfenweise Zugabe zu konzentrierter Schwefelsäure unter Kühlung auf 0°C - 10°C, und Halten der Reaktion 3-6 Stunden lang bei 0°C - 10°C; Waschen des festen Filterkuchens nach Saugfiltration mit Wasser, Lösen mit 0,27X Natriumhydroxidlösung; und Filtrieren unter Absaugen; Einstellen des Filtrats mit 5-10 %iger Salzsäure auf einen pH-Wert von 1-2; und Filtrieren; Waschen des Filterkuchens mit Wasser bis das Filtrat neutral ist; und Trocknen bei 40°C - 50°C, um Adamantan-Säure zu erhalten.

4. Verfahren nach Anspruch 1, wobei Schritt (3) weiterhin umfasst: Kühlen von konzentrierter Schwefelsäure auf 0°C - 10°C und Zugeben von Adamantansäure unter Rühren; langsames tropfenweises Zugeben von Salpetersäure und anschließendes tropfenweise Zugeben von Acetonitril und Halten der Reaktion 2-3 Stunden lang bei 0°C - 10°C; Überführen der Reaktionslösung in Eiswasser und Rühren der Reaktionslösung 16 bis 18 Stunden lang; anschließendes Filtrieren unter Absaugen; und Waschen des Filterkuchens mit Wasser; und Trocknen bei 40°C bis 50°C, um Amidoadamantansäure zu erhalten.

5. Verfahren nach Anspruch 1, wobei Schritt (4) weiterhin umfasst: Zugeben des Produkts aus Schritt (3) zu auf 0°C - 10°C abgekühltem Tetrahydrofuran; und Zugeben von Triethylamin in mehreren Portionen; anschließend tropfenweises Zugeben von Ethylchlorformiat und Durchführen der Reaktion bei Raumtemperatur für 4-6 Stunden; Filtrieren der resultierenden Reaktionsmischung und Waschen des Filterkuchens mit Tetrahydrofuran; Kombinieren der Tetrahydrofuranphasen und Abkühlen auf 0°C - 10°C; Zugeben von Natriumborhydrid in mehreren Portionen; anschließendes tropfenweise Zugeben von 0,8-1X Wasser und Durchführen der Reaktion für 2-3 Stunden; Zugeben von 5X Wasser; Filtrieren der Reaktionsmischung; Zentrifugaltrocknen des Filtrats; anschließendes Extrahieren mit Ethylacetat; und Trocknen der vereinigten Ethylacetatphasen über Natriumsulfat; Filtrieren und Zentrifugieren; und Versetzen des Rückstands mit 1,5-2,5X Ethylacetat; Rühren; Zugeben der gleichen Menge an Petrolether; und Rühren für 12-16 Stunden; Filtrieren; und Trocknen des Filterkuchen bei 40°C - 50°C, um ein Amidoadamantanol zu erhalten.

6. Verfahren nach Anspruch 1, wobei Schritt (5) folgende Reaktionen umfasst:
(5a) Hydrolyse: Sequenzielles Hinzufügen von Amidoadamantanol und einer starken Base zu Polyethylenglykol mit einem 5-fachen bis 10-fachen Volumen; Umsetzen durch Hochtemperaturhydrolyse bei 100°C - 180°C für 10 bis 18 Stunden; Abkühlen des Reaktionssystems; Verdünnen mit gereinigtem Wasser unter gleichmäßigem Rühren; Extrahieren der Wasserphase mit Toluol; Trocknen über Natriumsulfat und Filtrieren; Konzentrieren des Filtrats; und
(5b) Kondensation: Lösen des in Reaktion (5a) erhaltenen Produkts in Dichlormethan; Zugeben von Boc-Anhydrid; anschließendes Durchführen der Reaktion durch Kondensation bei 20°C - 30°C für 1-5 Stunden; und Konzentrieren der resultierenden Mischung, um Dichlormethan zu entfernen.

7. Verfahren nach Anspruch 1, wobei Schritt (6) weiterhin umfasst: Zugeben von n-Hexan zu der resultierenden restlichen öligen Flüssigkeit aus Schritt (5), die 2-3 Stunden lang durch Kristallisation umgesetzt wurde; und Zentrifugieren der resultierenden Mischung; Waschen mit n-Hexan und anschließendes Trocknen bei 40°C - 50°C für 16-18 Stunden, um Boc-geschütztes Amantadinol zu erhalten.

8. Verfahren nach Anspruch 1, wobei Schritt (7) weiterhin umfasst: Mischen von rauchender Salpetersäure mit Essigsäureanhydrid bei einer Temperatur von -10°C bis 10°C, um ein Nitrierreagenz zu erhalten; Lösen des aus Schritt (6) resultierenden festen Produkts in Dichlormethan bei einer Temperatur von -10°C bis 10°C; tropfenweises Zugeben des Nitrierungsreagenzes, das 15 Minuten bis 6 Stunden lang reagieren soll; anschließendes Abkühlen der Reaktion nach deren Beendigung mit Eiswasser und Trennen der Wasserphase; Waschen der organischen Phase mit dem 10-fachen Volumen einer Lösung von gesättigtem Natriumbicarbonat und einer Lösung von gesättigtem Natriumchlorid; Trocknen über Natriumsulfat; Filtrieren und Konzentrieren, um Dichlormethan zu entfernen, um ein Rohprodukt von Boc-geschütztem Amantadinnitrat zu erhalten.

9. Verfahren nach Anspruch 1, wobei Schritt (8) weiterhin umfasst: Lösen des Rohprodukts aus Schritt (7) in Alkohol; Zugeben von Wasser zur Kristallisation mit 2-3-stündigem Zerkleinern; anschließendes Filtrieren der resultierenden Mischung und Trocknen des resultierenden Feststoffs bei 40°C - 50°C, um ein aufgereinigtes Produkt zu erhalten.

10. Verfahren nach Anspruch 1, wobei Schritt (9) weiterhin umfasst: Lösen des aufgereinigten Produkts aus Schritt (8) in Ethylacetat bei einer Temperatur von 10°C bis 30°C, Zugeben von HCl/Ethylacetat; Durchführen der Reaktion für 16 bis 18 Stunden und anschließendes Filtrieren der resultierenden Mischung unter Absaugen und Trocknen, um Amantadinnitrat-Hydrochlorid zu erhalten.

11. Verfahren nach Anspruch 1, wobei Schritt (10) weiterhin umfasst: Zugeben des Produkts aus Schritt (9) in Ethanol; Behandeln durch Filtration, wobei das Filtrat konzentriert wird; und Zugeben von Ethylacetat zur Kristallisation unter Rühren; Trocknen der resultierenden Mischung bis zu einem konstanten Gewicht, um ein gereinigtes Amantadinhydrochloridnitrat zu erhalten.

12. Verfahren nach Anspruch 2, wobei bei der Reaktion (1a) der Bromierung in Schritt (1) das Molverhältnis der eingesetzten Reaktionskomponenten wie folgt ist: Rohmaterial: Brom = 1 : 2-6, die Reaktionstemperatur 60°C - 90°C beträgt und die Reaktionszeit 4 bis 6 Stunden beträgt; wobei bei der Reaktion (1b) der Hydrolyse in Schritt (1) das Molverhältnis der eingesetzten Reaktionskomponenten: Rohmaterial: Natriumoxalat = 1 : 2,5-6,0 beträgt, die verwendete Wassermenge 23-35X beträgt und die Reaktionszeit 2 bis 3 Stunden beträgt; oder wobei in Schritt (1) der Aufarbeitungsprozess umfasst: Zugeben von 3-5X Natriumsulfitlösung zur Reaktionslösung; Rühren bei Raumtemperatur für 16-18 Stunden und anschließendes Filtrieren; Waschen des Filterkuchen mit Wasser und anschließendes Lösen mit 3-8X organischem Lösungsmittel; Trocknen über wasserfreiem Natriumsulfat; und Filtrieren und Waschen des Filterkuchens mit 2-5X organischem Lösungsmittel; Zentrifugieren unter vermindertem Druck, um Adamantanol als Feststoff zu erhalten.

13. Verfahren nach Anspruch 3, wobei in Schritt (2) das Molverhältnis der eingesetzten Reaktionskomponenten wir folgt ist: Rohmaterial: Ameisensäure : Schwefelsäure = 1 : 6-10 : 11-16.

14. Verfahren nach Anspruch 1, wobei in Schritt (3) das Molverhältnis der eingesetzten Reaktionskomponenten: Rohmaterial wie folgt ist: Salpetersäure : Schwefelsäure : Acetonitril = 1 : 1,6-2,5 : 9-15 : 1,1-1,5; oder wobei in Schritt (4) das Molverhältnis der eingesetzten Reaktionskomponenten wie folgt ist: Rohmaterial : Triethylamin : Ethylchlorformiat : Natriumborhydrid = 1 : 1,2-1,5 : 1,2-1,5 : 1,5-2,5.

15. Verfahren nach Anspruch 6, wobei bei der Hydrolyse in Schritt (5) die starke Base Lithiumhydroxid, Natriumhydroxid oder Kaliumhydroxid ist und das Molverhältnis der eingesetzten Reaktionskomponenten wie folgt ist: Rohmaterial : Base = 1 : 5-10, wobei die Reaktionstemperatur 100°C - 180°C beträgt, die Reaktionszeit 15-16 h beträgt; oder wobei bei der Kondensation in Schritt (5) die Menge an Dichlormethan das 5- bis 10-fache des Volumens des Rohmaterials ist und das Molverhältnis der eingesetzten Reaktionskomponenten: Rohmaterial : Boc-Anhydrid = 1 : 1,05-1,5 beträgt und die Reaktionszeit 1-5 h beträgt.

16. Verfahren nach Anspruch 7, wobei in Schritt (6) die zur Kristallisation verwendete Menge an n-Hexan das 5- bis 9-fache des Volumens des Rohmaterials beträgt.

17. Verfahren nach Anspruch 8, wobei in Schritt (7) das Molverhältnis der Reaktanten: Rohmaterial : Essigsäureanhydrid : rauchende Salpetersäure = 1 : 1,08-1,8 : 1,68-2,8 beträgt; oder wobei das Nitrierungsmittel bei -10°C - 10°C hergestellt und 0,5-1 h gerührt wird; und wobei die Reaktionstemperatur 0°C - 10°C beträgt und die Reaktionszeit 15 min bis 6 h oder 30 min bis 3 h beträgt.

18. Verfahren nach Anspruch 9, wobei in Schritt (8) der zur Aufreinigung verwendete Alkohol Methanol oder Ethanol ist und das Gewichtsverhältnis der eingesetzten Reaktionskomponenten: Rohmaterial : Alkohol : Wasser = 1 : 2-5 : 3-8 beträgt; wobei, nachdem das Rohprodukt aus Schritt (7) vollständig in Alkohol gelöst ist, ein Teil des Wassers zugegeben und gerührt wird, bis sich ein weißer Feststoff absetzt, dann weitere 0,5-1 h gerührt wird und der verbleibende Teil des Wassers unter 1-2-stündigem Rühren zugegeben wird; und wobei die Temperatur für die Kristallisation auf 20°C - 30°C gehalten wird.

19. Verfahren nach Anspruch 10, wobei in Schritt (9) die Konzentration von HCl in HCl/Ethylacetat im Bereich von 2,0 bis 4,37 M liegt, das Molverhältnis der eingesetzten Reaktionskomponenten: Rohmaterial : HCl = 1 : 5 bis 10 beträgt, die Reaktionstemperatur 20°C bis 30°C beträgt und die Reaktionszeit 16 bis 18 Stunden beträgt; und wobei das aufgereinigte Produkt aus Schritt (8) zunächst in der 3- bis 6-fachen Menge an Ethylacetat gelöst wird und anschließend HCl/Ethylacetat zur Reaktion hinzugefügt wird.

20. Verfahren nach Anspruch 11, wobei Schritt (10) weiterhin umfasst: Lösen des Produkts aus Schritt (9) in Alkohol und Konzentrieren unter vermindertem Druck, um 0,5-1X Ethanol im System zu belassen; anschließendes Zugeben von Ethylacetat unter Rühren für 2-3 h, wobei das Gewichtsverhältnis der eingesetzten Reaktionskomponenten: Rohmaterial : Ethanol : Ethylacetat = 1 : 2,5-3 : 15-25 beträgt, die Temperatur der Konzentration mit Ethanol 40°C - 50°C beträgt und die Temperatur zum Trocknen 45°C - 50°C beträgt.

## Revendications

1. Procédé de fabrication de dérivés de nitrate d'amantadine, comprenant les étapes suivantes : (1) synthèse d'adamantanol à partir d'adamantane ; (2) carboxylation de l'adamantanol ; (3) acétylation de l'acide adamantanoïque ; (4) réduction ; (5) hydrolyse de l'amido-adamantanol et protection du groupe amino par Boc ; (6) cristallisation de l'amantadinol protégé par Boc ; (7) estérification en nitrate de l'amantadinol protégé par Boc ; (8) raffinage du produit de l'estérification en nitrate ; (9) déprotection du Boc et formation de sel ; et (10) raffinage du chlorhydrate de nitrate d'amantadine : 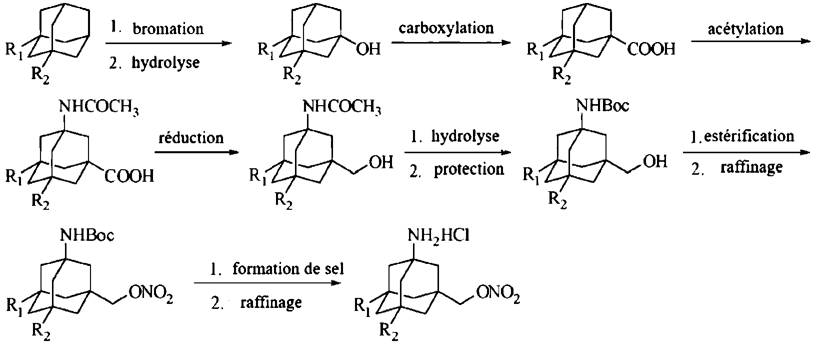
dans lequel l'adamantane a la structure de : 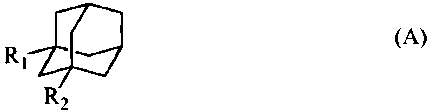
dans lequel R₁ et R₂ sont chacun indépendamment un atome d'hydrogène, un groupe alkyle à chaîne linéaire ou à chaîne ramifiée, un groupe aryle ou hétéroaryle substitué ou non substitué ;
dans lequel les étapes comprennent en outre :
(1) synthèse de l'adamantanol : traitement d'un adamantane substitué ou non substitué à bromer avec du brome liquide et hydrolyser avec de l'oxalate de sodium pour former un adamantanol ;
(2) carboxylation de l'adamantanol : traitement de l'adamantanol par réaction de Koch-Haff, et condensation avec de l'acide formique pour obtenir un acide adamantanoïque ;
(3) acétylation de l'acide adamantanoïque : traitement de l'acide adamantanoïque par condensation avec de l'acétonitrile par réaction de Ritter pour obtenir un acide amido-adamantanique ;
(4) réduction : traitement de l'acide amido-adamantanique par réduction avec du borohydrure de sodium pour obtenir un amido-adamantanol ;
(5) hydrolyse de l'amido-adamantanol et protection du groupe amino par Boc : traitement de l'amido-adamantanol, après élimination d'un groupe acyle par hydrolyse avec un alcali fort, par condensation avec de l'anhydride de Boc ;
(6) cristallisation de l'amantadinol protégé par Boc : traitement du produit de condensation par Boc par cristallisation avec macération dans du n-hexane ;
(7) estérification en nitrate de l'amantadinol protégé par Boc : réaction de l'amantadinol protégé par Boc avec un agent de nitration à une température de -10 °C à +10 °C pour obtenir un produit d'estérification en nitrate, dans lequel l'agent de nitration est un réactif avec un mélange d'acide nitrique fumant et d'anhydride acétique ;
(8) raffinage du produit d'estérification en nitrate : dissolution du produit d'estérification en nitrate dans un alcool, et ajout d'eau pour la cristallisation avec macération ;
(9) déprotection du Boc et formation de sel : élimination du groupe protecteur Boc du produit obtenu à l'étape (8) par hydrolyse avec de l' HCl pour obtenir un chlorhydrate de nitrate d'amantadine ; et
(10) raffinage du chlorhydrate de nitrate d'amantadine : dissolution du chlorhydrate de nitrate d'amantadine dans de l'éthanol, la solution résultante étant filtrée, concentrée et soumise à évaporation pour éliminer partiellement l'éthanol, ajout d'acétate d'éthyle pour la cristallisation avec macération, pour obtenir, après centrifugation, un chlorhydrate de nitrate d'amantadine ;
dans lequel, à l'étape (5), le solvant de réaction pour l'hydrolyse est le polyéthylène glycol 400 ; et à l'étape (7), le rapport molaire de l'alimentation de réaction est matière première: anhydride acétique:acide nitrique fumant = 1:1,08 à 1,8:1,68 à 2,8 ; et à l'étape (9), le rapport molaire de l'alimentation de réaction est matière première:HCl = 1:5 à 10, la température de réaction est de 20 °C à 30 °C et le temps de réaction est de 16 à 18 heures.

2. Procédé selon la revendication 1, dans lequel l'étape (1) comprend en outre :
(1a) bromation : réaction d'un adamantane substitué ou non substitué avec du brome liquide au reflux pendant 4 à 6 h pour obtenir un bromoadamantane ; et
(1b) hydrolyse : ajout d'oxalate de sodium et d'eau, le système réactionnel étant porté au reflux à 75 °C par hydrolyse pour obtenir un adamantanol substitué ou non substitué.

3. Procédé selon la revendication 1, dans lequel l'étape (2) comprend en outre : le produit de l'étape (1) étant dissous dans de l'acide formique, et ajouté lentement goutte à goutte à de l'acide sulfurique concentré avec un refroidissement à 0 à 10 °C, puis la réaction étant maintenue à 0 à 10 °C pendant 3 à 6 h ; la solution réactionnelle étant versée dans de l'eau glacée et agitée pendant 16 à 18 h ; après filtration par aspiration, le gâteau de filtration solide étant lavé avec de l'eau, dissous avec une solution de 0,27 X d'hydroxyde de sodium et filtré par aspiration ; le filtrat étant acidifié avec de l'acide chlorhydrique à 5 à 10 % jusqu'à pH = 1 à 2 et filtré, le gâteau de filtration étant lavé avec de l'eau jusqu'à ce que le filtrat soit neutre et séché à 40 à 50 °C pour obtenir l'acide adamantanoïque.

4. Procédé selon la revendication 1, dans lequel l'étape (3) comprend en outre : le refroidissement d'acide sulfurique concentré à 0 à 10 °C, et l'ajout d'acide adamantanoïque à dissoudre sous agitation, l'ajout lent goutte à goutte d'acide nitrique, puis l'ajout goutte à goutte d'acétonitrile, la réaction étant maintenue à 0 à 10 °C pendant 2 à 3 h ; la solution réactionnelle étant versée dans de l'eau glacée sous agitation pendant 16 à 18 h et filtrée par aspiration, et le gâteau de filtration étant lavé avec de l'eau et séché à 40 à 50 °C pour obtenir l'acide amido-adamantanique.

5. Procédé selon la revendication 1, dans lequel l'étape (4) comprend en outre : l'ajout du produit de l'étape (3) à du tétrahydrofurane, refroidi à 0 à 10 °C, et l'ajout de triéthylamine par lots, puis l'ajout goutte à goutte de chloroformiate d'éthyle, et la réaction étant réalisée à température ambiante pendant 4 à 6 h ; la filtration du mélange réactionnel résultant, et le lavage du gâteau de filtration avec du tétrahydrofurane, la combinaison des phases de tétrahydrofurane et le refroidissement à 0 à 10 °C, l'ajout de borohydrure de sodium par lots, puis l'ajout de 0,8 à 1X d'eau goutte à goutte, et la réaction étant réalisée pendant 2 à 3 h ; l'ajout de 5X d'eau, la filtration du mélange réactionnel, le filtrat étant séché par centrifugation, puis extrait avec de l'acétate d'éthyle, et les phases d'acétate d'éthyle combinées étant séchées sur du sulfate de sodium, filtrées et séchées par centrifugation, et le résidu étant ajouté avec 1,5 à 2,5X d'acétate d'éthyle, bien agité, la même quantité d'éther de pétrole étant ajoutée, et agité pendant 12 à 16 h, filtré et le gâteau de filtration étant séché à 40 à 50 °C pour obtenir un amido-adamantanol.

6. Procédé selon la revendication 1, dans lequel l'étape (5) comprend les réactions suivantes :
(5a) hydrolyse : ajout d'amido-adamantanol et d'une base forte de manière séquentielle à du polyéthylène glycol avec un volume 5 à 10 fois supérieur, réaction par hydrolyse à haute température à 100 à 180 °C pendant 10 à 18 h, et le système réactionnel étant refroidi, dilué avec de l'eau purifiée et agité uniformément, et la phase aqueuse étant extraite avec du toluène, séchée sur du sulfate de sodium et filtrée, et le filtrat étant concentré ; et
(5b) condensation : dissolution du produit obtenu lors de la réaction (5a) dans du dichlorométhane, ajout d'anhydride de Boc, puis réalisation de la réaction par condensation à 20 à 30 °C pendant 1 à 5 h, et le mélange résultant étant concentré pour éliminer le dichlorométhane.

7. Procédé selon la revendication 1, dans lequel l'étape (6) comprend en outre : l'ajout de n-hexane au liquide huileux résiduel résultant de l'étape (5), la réaction par cristallisation pendant 2 à 3 h, et le mélange résultant étant centrifugé, lavé avec du n-hexane, puis séché à 40 à 50 °C pendant 16 à 18 h pour obtenir un amantadinol protégé par Boc.

8. Procédé selon la revendication 1, dans lequel l'étape (7) comprend en outre : le mélange d'acide nitrique fumant avec de l'anhydride acétique à une température de - 10 °C à 10 °C pour obtenir un réactif de nitration ; dissolution du produit solide résultant de l'étape (6) dans du dichlorométhane à une température régulée à -10 °C à 10 °C, ajout goutte à goutte du réactif de nitration à faire réagir pendant 15 min à 6 h, puis neutralisation de la réaction une fois achevée avec de l'eau glacée et la phase aqueuse étant séparée, et la phase organique étant lavée avec respectivement 10 volumes (10 vol) d'une solution saturée de bicarbonate de sodium et d'une solution saturée de chlorure de sodium, séchée sur du sulfate de sodium, filtrée et concentrée pour éliminer le dichlorométhane pour obtenir un produit brut de nitrate d'amantadine protégé par Boc.

9. Procédé selon la revendication 1, dans lequel l'étape (8) comprend en outre : la dissolution du produit brut de l'étape (7) dans un alcool, l'ajout d'eau pour la cristallisation avec macération pendant 2 à 3 h, puis le mélange résultant étant filtré, et le solide résultant étant séché à 40 à 50 °C pour obtenir un produit raffiné.

10. Procédé selon la revendication 1, l'étape (9), comprend en outre : la dissolution du produit raffiné de l'étape (8) dans de l'acétate d'éthyle à une température régulée de 10 à 30 °C, l'ajout d'un mélange HCl/acétate d'éthyle, la réalisation de la réaction pendant 16 à 18 h, puis le mélange résultant étant filtré par aspiration et séché pour obtenir un chlorhydrate de nitrate d'amantadine.

11. Procédé selon la revendication 1, l'étape (10) comprend en outre : l'ajout du produit de l'étape (9) dans de l'éthanol, le traitement par filtration avec concentration du filtrat, et l'ajout d'acétate d'éthyle pour la cristallisation sous agitation, le mélange résultant étant séché jusqu'à un poids constant pour obtenir un chlorhydrate de nitrate d'amantadine raffiné.

12. Procédé selon la revendication 2, dans lequel, dans la réaction de bromation (1a) de l'étape (1), le rapport molaire de l'alimentation de réaction est matière première:brome = 1:2 à 6, la température de réaction est de 60 °C à 90 °C, et le temps de réaction est de 4 à 6 h ; dans lequel, dans la réaction d'hydrolyse (1b) de l'étape (1), le rapport molaire de l'alimentation de réaction est matière première: oxalate de sodium = 1:2,5 à 6,0, la quantité d'eau utilisée est de 23 à 35X, et le temps de réaction est de 2 à 3 h ; ou dans lequel, à l'étape (1), le procédé de post-traitement comprend : l'ajout de 3 à 5X d'une solution de sulfite de sodium à la solution réactionnelle, l'agitation à température ambiante pendant 16 à 18 h, puis la filtration, et le gâteau de filtration étant lavé avec de l'eau, puis dissous avec 3 à 8X d'un solvant organique, séché sur du sulfate de sodium anhydre et filtré, et le gâteau de filtration étant lavé avec 2 à 5X d'un solvant organique et séché par centrifugation sous pression réduite pour obtenir l'adamantanol sous forme de solide.

13. Procédé selon la revendication 3, dans lequel, à l'étape (2), le rapport molaire de l'alimentation de réaction est matière première:acide formique:acide sulfurique = 1:6 à 10:11 à 16.

14. Procédé selon la revendication 1, dans lequel, à l'étape (3), le rapport molaire de l'alimentation de réaction est matière première:acide nitrique:acide sulfurique: acétonitrile = 1:1,6 à 2,5:9 à 15:1,1 à 1,5, ou dans lequel, à l'étape (4), le rapport molaire de l'alimentation de réaction est matière première:triéthylamine:chloroformiate d'éthyle:borohydrure de sodium= 1:1,2 à 1,5:1,2 à 1,5:1,5 à 2,5.

15. Procédé selon la revendication 6, dans lequel, dans l'hydrolyse de l'étape (5), la base forte est l'hydroxyde de lithium, l'hydroxyde de sodium ou l'hydroxyde de potassium, et le rapport molaire de l'alimentation de réaction est matière première:base = 1:5 à 10 ; dans lequel la température de réaction est de 100 à 180 °C, le temps de réaction est de 15 à 16 h ; ou dans lequel, dans la condensation de l'étape (5), la quantité de dichlorométhane est de 5 à 10 fois le volume de la matière première, et le rapport molaire de l'alimentation de réaction est matière première:anhydride de boc = 1:1,05 à 1,5, et le temps de réaction est de 1 à 5 h.

16. Procédé selon la revendication 7, dans lequel à l'étape (6), la quantité de n-hexane utilisée pour la cristallisation est 5 à 9 fois le volume de la matière première.

17. Procédé selon la revendication 8, dans lequel à l'étape (7), le rapport molaire des réactifs est matière première:anhydride acétique:acide nitrique fumant = 1:1,08 à 1,8:1,68 à 2,8 ; ou dans lequel le réactif de nitration est préparé à -10 à 10 °C et agité pendant 0,5 à 1 h ; et dans lequel la température de réaction est de 0 à 10 °C, et le temps de réaction est de 15 min à 6 h, ou de 30 min à 3 h.

18. Procédé selon la revendication 9, dans lequel à l'étape (8), l'alcool utilisé pour le raffinage est le méthanol ou l'éthanol, et le rapport pondéral de l'alimentation de réaction est matière première:alcool:eau = 1:2 à 5:3 à 8 ; dans lequel, une fois que le produit brut de l'étape (7) est complètement dissous dans l'alcool, une partie de l'eau est ajoutée, et le mélange est agité jusqu'à la précipitation d'un solide blanc, puis davantage agité pendant 0,5 à 1 h, et la partie restante de l'eau est ajoutée sous agitation pendant 1 à 2 h ; et dans lequel la température pour la cristallisation est régulée à 20 à 30 °C.

19. Procédé selon la revendication 10, dans lequel à l'étape (9), la concentration en HCl dans le mélange HCl/acétate d'éthyle est comprise dans la plage de 2,0 à 4,37 M, le rapport molaire de l'alimentation de réaction est matière première:HCl = 1:5 à 10, la température de réaction est de 20 à 30 °C, et le temps de réaction est de 16 à 18 h ; et dans lequel le produit raffiné de l'étape (8) est d'abord dissous dans 3 à 6 fois le poids d'acétate d'éthyle, puis un mélange HCl/acétate d'éthyle est ajouté pour la réaction.

20. Procédé selon la revendication 11, dans lequel l'étape (10) comprenant en outre : la dissolution du produit de l'étape (9) dans un alcool, et la concentration sous pression réduite pour maintenir 0,5 à 1 fois la quantité d'éthanol dans le système, puis l'ajout d'acétate d'éthyle avec macération pendant 2 à 3 h, et dans lequel le rapport pondéral de l'alimentation de réaction est matière première:éthanol:acétate d'éthyle = 1:2,5 à 3:15 à 25, la température de concentration avec de l'éthanol est de 40 à 50 °C, et la température pour le séchage est de 45 à 50 °C.
